# EUROPEAN PATENT APPLICATION

(11) **EP 4 345 106 A1**
(43) Date of publication of application: **03.04.2024**
(21) Application number: 22199230.8
(22) Date of filing: 30.09.2022
(51) Int. Cl.: C07K 14/665, A61P 25/08, A61P 43/00, C07K 14/70, C12N 15/85

(54) **GENE THERAPY VECTORS FOR THE EXPRESSION OF PREPRODYNORPHIN VARIANTS FOR THE TREATMENT OF EPILEPSY**

(71) Applicant: Charité - Universitätsmedizin Berlin, 10117 Berlin (DE)
(72) Inventor: The designation of the inventor has not yet been filed
(74) Representative: Kilger, Ute

(57) **Abstract**

Subject matter of the present invention is a delivery vector comprising a DNA sequence encoding pre-prodynorphin or pre-prodynorphin-variants and wherein said delivery vector drives expression of a pre-propeptide in a target cell, and wherein said delivery vector comprising said DNA sequence enables the release of dynorphin or dynorphin-variants from the target cell on demand, and wherein said pre-propeptide is pre-prodynorphin or a pre-prodynorphin-variant and wherein said pre-propeptide comprises a signal peptide, wherein the signal peptide is a N-terminal extension of nascent polypeptide chains and wherein said signal peptide mediates protein targeting to the lumen of the endoplasmatic reticulum, and, wherein said pre-propeptide comprises a N-terminal pro-peptide fragment on the C-terminal end of said signal peptide and wherein said N-terminal pro-peptide fragment i) comprises a sorting motif consisting of the amino acid sequence DLXₓEX_{y}L or comprises ii) a sorting motif of a pre-pro-neuropeptide or protein sorted to large dense core vesicles, other than pre-prodynorphin, as defined herein, where x is an integer from 1 to 20, y is an integer from 1 to 10, and each instance of X may independently be any amino acid, and wherein said N-terminal pro-peptide fragment consists of 16 to 90 amino acids.

## Description

The present invention provides delivery vectors for transferring a nucleic acid sequence that encodes a pre-propeptide to a cell in vitro, ex vivo or in vivo. The present invention provides methods of delivering a nucleic acid sequence to a cell and methods of treating focal epilepsies.

With a prevalence of 1-2%, epilepsies belong to the most frequent neurological diseases worldwide (McNamara et al., 1999) with focal epilepsy accounting for around 70% of cases. Of these, mesial temporal lobe epilepsy (mTLE) is the most frequent clinical presentation. In mTLE the focus lies in or near the hippocampus where learning, memory and emotional control are modulated. mTLE represents an acquired disease frequently induced by traumatic brain injury. Also, CNS infections, high fevers, brain tumours, or vascular malformations can lead to epileptic foci. With ongoing disease, hippocampal sclerosis with accompanying neurological deficits may develop. These represent key clinical features of this subtype of mTLE (for review, see Engel et al., 2001). Despite the introduction of a large variety of antiepileptic drugs over the last few decades, the rate of drug-resistant epilepsies (30% to 70%) has not improved since the early study of Coatsworth in 1971 (Coatsworth et al., 1971, Loscher et al., 2011). To date, surgical resection of the epileptogenic focus remains as the ultimate treatment option for patients in whom the epileptic focus can be clearly defined and can be separated well from other critical CNS regions. The speech centre near to the hippocampal focus represents a major contraindication for epilepsy surgery. But even if epilepsy surgery can be performed, there is no guarantee for lasting seizure freedom. Only up to 50% of patients remain seizure-free for at least one year after removal of the epileptic focus (Spencer et al., 2008).

Since the early 1980s, there has been evidence that opioids, namely dynorphins (Dyn), act as modulators of neuronal excitability in vitro (Henriksen et al., 1982, Siggins et al., 1986). In line with this, the deletion of the prodynorphin (pDyn) coding sequence in mice (Loacker et al., 2007) and the finding of low Dyn levels in humans due to mutations in the promoter region of the pDyn gene (Stogmann et al., 2002, Gambardella et al., 2003) are associated with increased vulnerability to the development of epilepsy. In most animal models of temporal lobe epilepsy (TLE; comprising epilepsies arising in the temporal lobe = lateral TLE and mTLE), cortical and hippocampal pDyn gene expression is reduced after an initial, short peak of over-expression (for review, see (Simonato et al., 1996, Schwarzer et al., 2009). This finding is in line with the description of assumedly short-lived, post-ictally increased pDyn mRNA levels in hippocampal granule cells (Pirker et al., 2009). In an earlier study, an overall reduction of Dyn-immunoreactivity in surgically removed brain tissue obtained from mTLE patients had been described (de Lanerolle et al., 1997).

Dynorphins act preferentially on kappa opioid receptors (KOR). Despite the reduction of endogenous Dyn, KOR remains available as drug target under epileptic conditions, and the application of KOR agonistic drugs can suppress experimental seizures (Tortella et al., 1988, Takahashi et al., 1990, Solbrig et al., 2006, Loacker et al., 2007, Zangrandi et al. 2016). Various selective KOR agonists applied through different routes of administration yielded time- and dose-dependent effects similar to those upon treatment with phenytoin or phenobarbital in models of epilepsy (for review, see (Simonato et al., 1996). We previously demonstrated that activation of KOR promotes the survival of hippocampal neurons subsequent to the acute epileptic phase after unilateral injection of kainic acid in mice (Schunk et al., 2011).

The object of the present invention is to provide delivery vectors to transfer a nucleic acid sequence encoding a pre-propeptide or a peptide comprising a sequence that enables the packing of said pro-peptide, e.g. a prodynorphin into vesicles, wherein the pro-peptide undergoes maturation and the active substance which is a dynorphin or variant of dynorphin is released upon a series of action potentials that exceed a certain excitation threshold. The object of the present invention is, thus, to provide delivery vectors for transferring a nucleic acid sequence to a cell in vitro, ex vivo or in vivo. Object of the invention is in particular a vector-based therapy for treatment of focal epilepsies with pre-prodynorphin or dynorphin or variants thereof. The inventive delivery vectors comprising a nucleic acid encoding pre-prodynorphin or prodynorphin or dynorphin or variants thereof shall transduce neurons, express, process, store and release pre-prodynorphin or prodynorphin or dynorphin or variants thereof and thus provide activation of KOR in the epileptogenic focus, thereby inhibiting seizures.

It is an aim of the invention to further develop the AAV-based gene vector for the expression of pre-prodynorphin for the therapy of focal epilepsy. Here, AAV vectors are preferably used for the expression of pre-prodynorphin. In the present invention, following vector transduction of neurons, pre-prodynorphin is expressed and processed to dynorphin peptides (dyn) of different types and lengths, which are released after stimulation by the trigger, which is highfrequency excitation.

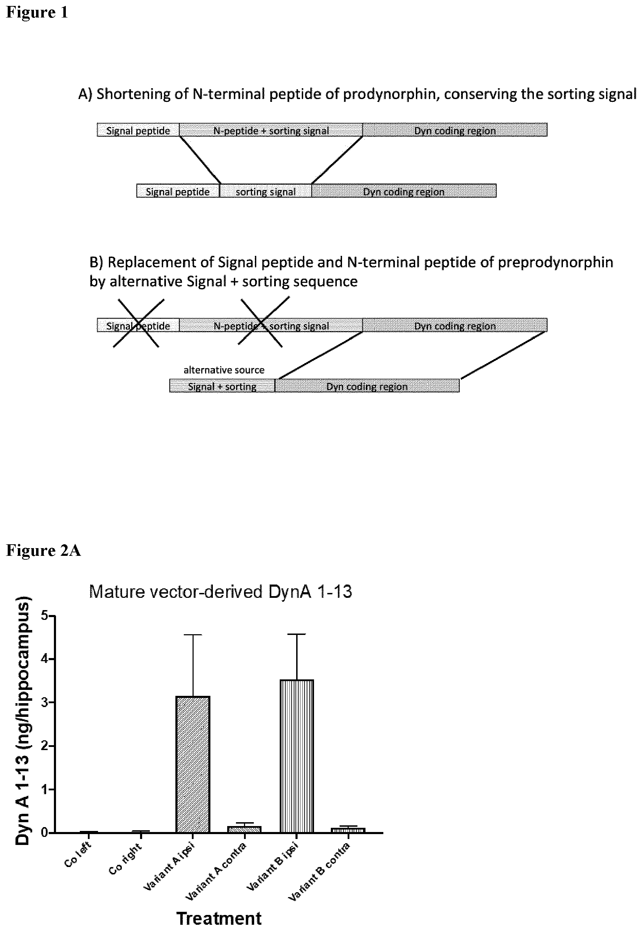

The present invention is further based on the truncation of the ppDYN protein. In order to avoid interfering with the correct vesicular targeting in neurons or with the correct processing into defined peptides, two strategies were used:
First, the region of the so-called N-peptide of ppDyn was shortened. Here, it was found that this region contains a specific sorting motif which was maintained. Secondly, for some neuropeptides (e.g. pPOMC or ppEnk), these N-terminally located signal and sorting sequences are significantly shorter than the N-terminally located signal and sorting sequence of ppDyn. Therefore, fusion proteins that combine the N-terminal of part of such neuropeptide-precursor molecules stemming from pPOMC with the C-terminal sequence of ppDyn coding for the various active Dyn peptides were constructed (Fig.1).

### Detailed description of the invention

Subject matter of the present invention are delivery vectors comprising a DNA sequence encoding pre-prodynorphin or pre-prodynorphin-variants and
- wherein said delivery vector drives expression of a pre-propeptide in a target cell, and
- wherein said delivery vector comprising said DNA sequence enables the release of dynorphin or dynorphin-variants from the target cell on demand, and
- wherein said pre-propeptide is pre-prodynorphin or a pre-prodynorphin-variant and
- wherein said pre-propeptide comprises a signal peptide, wherein the signal peptide is a N-terminal extension of a nascent polypeptide chain and wherein said signal peptide mediates protein targeting to the lumen of the endoplasmatic reticulum, and,
- wherein said pre-propeptide comprises either (i) a N-terminal pro-peptide fragment on the C-terminal end of said signal peptide and wherein said N-terminal pro-peptide fragment comprises a sorting motif consisting of the amino acid sequence DLXₓEX_{y}L (SEQ ID No. 36), where x is an integer from 1 to 20, y is an integer from 1 to 10, and each instance of X may independently be any amino acid (for the avoidance of doubt, this means that in the present invention in the first sequence of e.g. 1 to 20 X, each X may individually be any amino acid, and in the second sequence of e.g. 1 to 10 X, each X may individually be any amino acid, particularly as further defined herein), or wherein said pre-propeptide comprises (ii) a N-terminal pro-peptide fragment of a pre-pro-neuropeptide or protein sorted to large dense core vesicles, other than pre-prodynorphin, on the C-terminal end of said signal peptide and wherein said N-terminal pro-peptide fragment comprises a sorting motif of said pre-pro-neuropeptide or protein sorted to large dense core vesicles, and wherein said N-terminal pro-peptide fragment consists of 16 to 90 amino acids, and
- wherein said pre-prodynorphyin or pre-prodynorphin-variants comprise at least one of the following sequences selected from the group consisting of a,b,c,d,e,f:
   a. Dyn A that is SEQ ID No. 2 (AA 207-223 of SEQ ID No. 1; ppDyn) or a variant thereof consisting of the first 13 AA (first from the N-terminal end) or a variant thereof consisting of the first 8 AA (first from the N-terminal end)
   b. Dyn B that is SEQ ID No. 3 (AA 226-238 of SEQ ID No. 1; ppDyn)
   c. leumorphin that is SEQ ID No. 4 (AA 226-254 of SEQ ID No. 1; ppDyn)
   d. variants of Dyn A according to SEQ ID No.2 having an amino acid sequence identity of at least 60 % within the first 8 AA from the N-terminal end of SEQ ID No. 2 (YGGFLRRI) i.e. having an amino acid sequence identity of at least 60 % within the sequence YGGFLRRI comprised in SEQ ID No. 2.
   e. variants of Dyn B according to SEQ ID No. 3 having an amino acid sequence identity of at least 60 % within the first 8 AA from the N-terminal end of SEQ ID No. 3 (YGGFLRRQ) i.e. having an amino acid sequence identity of at least 60 % within the sequence YGGFLRRQ comprised in SEQ ID No. 3.
   f. variants of leumorphin according to SEQ ID No. 4 having an amino acid sequence identity of at least 60 % within the first 8 AA from the N-terminal end of SEQ ID No. 4 (YGGFLRRQ), i.e. having an amino acid sequence identity of at least 60 % within the sequence YGGFLRRQ comprised in SEQ ID No. 4.

In this context, 60 % sequence identity is defined as follows: 3 of the first 8 N-terminal amino acids may be removed or replaced by another amino acid. Percentage of sequence identity is calculated for the shortened peptide in case of truncated peptide variants. Introduction of additional amino acids are handled as gap in the original sequence, deletions are handled as gap in the modified peptide for calculation of sequence identity (YGGFLRRQ differs from YG-FLRRQ only by 1 AA, although now AA in positions 3, 4, 5, 6 and 7 are different). In any case a variant of SEQ ID No. 2 having an amino acid sequence identity of at least 60 % from the N-terminal end in the first 8 AA may be a variant that comprises the sequence: YGZFLRKZ with each Z individually standing for any amino acid and K resubstituting R in position 7, conserving the peptidase recognition site (RK or RR).

In the present invention, "amino acids" stands for naturally occurring amino acids, which are more particularly the canonical amino acids, i.e. the amino acids that are encoded directly by the codons of the universal genetic code.

Throughout the present invention "Z" in an amino acid sequence stands for any of the naturally occurring amino acids, in a specific embodiment "Z" may be selected from the group comprising alanine, glycine, asparagine, glutamine, leucine, serine, valine and isoleucine.

Pre-pro-neuropeptides other than pre-prodynorphin are known to the person skilled in the art and are described e.g., in Zhang et al. Progress in neurobiology 90 (2010) 276-283:
A pre-pro-neuropeptide or protein sorted to large dense core vesicles other than pre-prodynorphin may be selected from the group comprising but not limited to pre-pro-Substance P, pPOMC, pptachykinin A, pptachykinin B, pproopiomelanocortin, ppcholecystokinin, ppchromogranin B, calcitonin gene-related peptide (CGRP), pre-proEnkephalin, pre-proBDNF, pre-proTachykinin, pre-pro-Somatostatin, pre-pro-VIP, pre-pro-CCK, pre-proNociceptin or pre-proNPY.

Pre-pro-neuropeptides or proteins sorted to large dense core vesicles including pre-prodynorphin have a signal peptide at their extreme N-terminus that directs them to translocate into the endoplasmic reticulum (ER) as above described. Neuropeptides are expressed in neurons and secreted in response to physiological or pathological stimuli which means that they are released on-demand. Neuropeptide prohormones exhibit a great diversity of sorting motifs. Some of them have a sorting motif consisting of the amino acid sequence DLXₓEX_{y}L (SEQ ID No. 36), where x is an integer from 1 to 20, y is an integer from 1 to 10, and each instance of X may independently be any amino acid as above described. Other pre-pro-neuropeptides, as e.g., ppneuropeptide Y, comprise another sorting motif than DLXₓEX_{y}L (SEQ ID No. 36). In any event sorting shall be comprised in said above-defined N-terminal pro-peptide fragment that consists of 16 to 90 amino acids.

According to the present invention said delivery vector comprises a DNA sequence encoding the pre-propeptide of dynorphin or dynorphin-variants. This means said vector comprises a DNA sequence encoding a signal peptide fused to the propeptide. As one aspect, the present invention provides delivery vectors for transferring a nucleic acid to a cell, the delivery vector comprising a segment encoding a signal peptide targeting the pre-propeptide to the lumen of endoplasmatic reticulum. The DNA sequence encoding the signal peptide may be a sequence according to SEQ ID No.: 11. In another aspect of the invention said delivery vector comprises a DNA sequence encoding a propeptide fragment. In a particular aspect of the invention said propeptide fragment is a sequence according to SEQ ID No. 5.

The advantage of the present delivery vectors is a release on demand of dynorphins or dynorphin-variants. Particularly, this means that prodynorphin (or a variant of prodynorphin) is packed into vesicles, undergoes maturation and is released on demand upon high frequency stimulation (e.g. stimulation ≥ 8 Hz) as it occurs at seizure onset. Particularly, a release-on-demand formulation, thus, provides a pre-prodynorphin (or a variant of pre-prodynorphin) that is then packed into vesicles, undergoes maturation and the active substance which is a dynorphin or variant of dynorphin is released upon a frequency of action potentials that exceeds a certain threshold. In other words, release of dynorphin or dynorphin-variants from the target cell "on demand" particularly denominates a "release upon high frequency stimulation" as it occurs at seizure onset and/or "release upon a frequency of action potentials that exceed a certain threshold". Said certain threshold may be a threshold that is ≥ 6 Hz, in another embodiment ≥ 7 Hz in another embodiment ≥ 8 Hz, in another embodiment ≥ 9 Hz. This means said release-on-demand is triggered by increased neuronal firing frequency. Said increased neuronal firing frequency may be measured by EEG (electroencephalography) as spike trains, "increased" means a frequency of spikes in a train measured by EEG in said subject that is ≥ 6 Hz, in another embodiment ≥ 7 Hz in another embodiment ≥ 8 Hz, in another embodiment ≥ 9 Hz.

This means that the present delivery vectors drive expression of pre-propeptides that enable the provision of dynorphin or dynorphin-variants on demand as such delivery vectors first express the pre-propeptides in neurons, where the resulting propeptides are sorted into large dense core vesicles, where they are enzymatically processed and the derived peptides are stored until a sufficiently intense excitation leads to their release, i.e. said release is triggered by increased neuronal firing frequency as explained above. Dyn peptides bind to pre- and/or postsynaptic KOR which activate G-proteins, which, beside others, regulate ion channels to dampen further amplification and spread of neuronal excitation. The translation of the signal peptide of ppDyn is the initial step, guiding ppDyn into the endoplasmatic reticulum, from where prodynorphin is sorted into "large dense core" vesicles (LDV). Using existing mechanisms in neurons, the prodynorphin is enzymatically processed to mature peptides and transported to axon terminals. LDV are stored in the axon terminals and released in a stimulation-dependent manner. High frequency stimulation as explained above, like at the onset of seizures, induce the release, while low-frequency stimulation does not. This creates a release on demand situation. Released Dyn peptides bind to pre- and/or postsynaptic KOR, which activate G-proteins, that, beside others, regulate ion channels to dampen further amplification and spread of neuronal excitation.

In other words, a release on demand composition is a composition that releases the peptide having agonistic effects on human KOR derived from any of the delivery vectors or recombinant virus particles or liposomes or nanoparticles according to the present invention at the onset of seizures in said subject. The onset of seizures. may be characterized by increased neuronal firing frequency that may be measured by EEG (electroencephalography) as spike trains, increased means a frequency of spikes in a train measured by EEG in said subject that is ≥ 6 Hz, in another embodiment ≥ 7 Hz in another embodiment ≥ 8 Hz, in another embodiment ≥ 9 Hz.

Subject matter of the present invention is a delivery vector comprising a DNA sequence encoding pre-prodynorphin or pre-prodynorphin-variants wherein said N-terminal pro-peptide fragment consists of 16 to 90 amino acids, preferably 20 to 90 amino acids, preferably 30 to 90 amino acids.

Subject matter of the present invention is a delivery vector comprising a DNA sequence encoding pre-prodynorphin or pre-prodynorphin-variants wherein said N-terminal pro-peptide fragment on the C-terminal end of the signal peptide is a modified propeptide fragment of ppDyn wherein the unmodified propeptide fragment of ppDyn is 
SEQ ID No. 5:
DCLSRCSLCA VKTQDGPKPI NPLICSLQCQ AALLPSEEWE RCQSFLSFFT
PSTLGLNDKE DLGSKSVGEG PYSELAKLSG SFLKELEKSK FLPSISTKEN
TLSKSLEEKL RGLSDGFREG AESELMRDAQ LNDGAMETGT LYLAEEDPKE QV
 and wherein the modification of said propeptide fragment of SEQ ID No. 5 is a shortening while maintaining the sorting motif that is consisting of the amino acid sequence DLXₓEX_{y}L (SEQ ID No. 36).

Subject matter of the present invention is a delivery vector comprising a DNA sequence encoding pre-prodynorphin or pre-prodynorphin-variants according to the present invention, wherein said N-terminal pro-peptide fragment on the C-terminal end of the signal peptide is a modified propeptide fragment of ppDyn that comprises or consists of SEQ ID No: 6 DCLSRCSLCAVKTQDGPKPINPLICSLQCQAALLPSEEWERCQSFLSFFTPSTLGLNDK E**DL**GSKSVG**E**GPYS**EL**AK**L**SGSFL***RK***EQVKR

Subject matter of the present invention is a delivery vector comprising a DNA sequence encoding pre-prodynorphin or pre-prodynorphin-variants wherein said N-terminal pro-peptide fragment on the C-terminal end of the signal peptide is a modified propeptide fragment of ppDyn that comprises or consists of
SEQ ID No: 7 
DLGSKSVGEG PYSELAKLSG SFLKELEKSK FLPSISTKEN TLSKSLEEKL
RGLSDGFREG AESELMRDAQ LNDGAMETGT LYLAEEDPKE QV
or SEQ ID No 8
   **DL**GSKSVGEG PYS**E**LAKLSG SFL***RK***E QV
or SEQ ID No 9
   **DL**GSKSVGEG PYS**E**LAK**L*RK***E QV
or SEQ ID No. 55
   **DL**GSKSVG**E**G PYSEL***RK***E QV.

An embodiment of the present invention is a delivery vector comprising a DNA sequence encoding pre-prodynorphin or pre-prodynorphin-variants wherein said N-terminal pro-peptide fragment on the C-terminal end of the signal peptide is a modified pro-peptide fragment of ppDyn that comprises or consists of
SEQ ID No.: 56
DLGSKSVGEG PYSELAKLSG SFLK KE QV

Another embodiment of the present invention is a delivery vector comprising a DNA sequence encoding pre-prodynorphin or pre-prodynorphin-variants wherein said N-terminal pro-peptide fragment on the C-terminal end of the signal peptide is a modified propeptide fragment of ppDyn that comprises or consists of
SEQ ID No.: 57
DLGSKSVGEG PYSEL AKL

Another embodiment of the present invention is a delivery vector comprising a DNA sequence encoding pre-prodynorphin or pre-prodynorphin-variants wherein said N-terminal pro-peptide fragment on the C-terminal end of the signal peptide is a modified propeptide fragment of ppDyn that comprises or consists of
SEQ ID No.: 58
**DL**GSKSVG**E**G PYSE**L**

Subject matter of the present invention is a delivery vector comprising a DNA sequence encoding pre-prodynorphin or pre-prodynorphin-variants wherein said N-terminal pro-peptide region on the C-terminal end of the signal peptide is a modified hybrid propeptide fragment of ppDyn wherein the unmodified propeptide fragment of ppDyn is 
SEQ ID No. 5
DCLSRCSLCA VKTQDGPKPI NPLICSLQCQ AALLPSEEWE RCQSFLSFFT
PSTLGLNDKE DLGSKSVGEG PYSELAKLSG SFLKELEKSK FLPSISTKEN
TLSKSLEEKL RGLSDGFREG AESELMRDAQ LNDGAMETGT LYLAEEDPKE QV
 and wherein the modification of said propeptide fragment of SEQ ID No. 5 is a replacement of parts of SEQ ID No. 5 with a propeptide of or a fragment of a propeptide of a neuropeptide, wherein the modified propeptide fragment of ppDyn i) comprises at least one sorting motif consisting of the amino acid sequence DLXₓEX_{y}L (SEQ ID No. 36) or ii) comprises a sorting motif of said pre-pro-neuropeptide or protein sorted to large dense core vesicles, other than pre-prodynorphin, as defined further herein. For the avoidance of doubt: the skilled person can readily acknowledge that in this specific context obviously the propeptide of or a fragment of a propeptide of a neuropeptide is different from the unmodified propeptide fragment of ppDyn of SEQ ID No. 5, since parts of the sequence is to be replaced.

Subject matter of the present invention is a delivery vector comprising a DNA sequence encoding pre-prodynorphin or pre-prodynorphin-variants wherein the modified propeptide fragment comprises SEQ ID No. 10:
MPRSCCSRSGALLLALLLQASMEVRGWCLESSQCQ**DL**TT**E**SN**L**LECIRACKP.

Subject matter of the present invention is a delivery vector comprising a DNA sequence encoding pre-prodynorphin or pre-prodynorphin-variants wherein the modified propeptide fragment comprises or consists of a propeptide fragment of pre-proEnkephalin, pre-proBDNF, pre-proTachykinin, pre-pro-Somatostatin, pre-pro-VIP, pre-pro-CCK, pre-proNociceptin or pre-proNPY, wherein said propeptide fragment comprises said sorting motif as described above or another sorting motif. For example for ppNPY a different sorting motif was proposed. In particular, subject matter of the present invention is a delivery vector comprising a DNA sequence encoding pre-prodynorphin or pre-prodynorphin-variants wherein the modified propeptide fragment is selected from the group comprising of any of: SEQ ID Nos: 37 to 52.

These DNA sequences may be selected from DNA sequences encoding for a polypeptide from the group comprising:
SEQ ID No. 37: pre-proenkephalin - N-peptide 
MARFLTLCTWLLLLGPGLLATVRAECSQDCATCSYRLVRPADINFLACVMECEGKLP
SLKIWETCKELLQLSKPELPQDGTSTLRENSKPEESHLLA
SEQ ID No. 38: pre-proenkephalin-pDyn Hybrid: 
MARFLTLCTWLLLLGPGLLATVRAECSQDCATCSYRLVRPADINFLACVMECEGKLP
SLKIWETCKELLQLSKPELPQDGTSTLRENSKPEESHLLAKRYGGFLRKYPKRSSEVA
GEGDGDSMGHEDLYKRYGGFLRRIRPKLKWDNQKRYGGFLRRQFKVVTRSQEDPN
AYSGELFDA
SEQ ID No. 39: pre-pro-NPY - N-peptide
   MLGNKRLGLSGLTLALSLLVCLGALAEA
SEQ ID No. 40: pre-pro-NPY-pDyn Hybrid: 
MLGNKRLGLSGLTLALSLLVCLGALAEAKRYGGFLRKYPKRSSEVAGEGDGDSMGH
EDLYKRYGGFLRRIRPKLKWDNQKRYGGFLRRQFKVVTRSQEDPNAYSGELFD
SEQ ID No. 41: pre-pro BDNF - N-peptide 
MTILFLTMVISYFGCMKAAPMKEANIRGQGGLAYPGVRTHGTLESVNGPKAGSRGLT
SLADTFEHVIEELLDEDHKVRPNEENNKDADLYTSRVMLSSQVPLEPPLLFLLEEYKN
YLDAANMSM
SEQ ID No. 42: pre-pro BDNF-pDyn Hybrid: 
MTILFLTMVISYFGCMKAAPMKEANIRGQGGLAYPGVRTHGTLESVNGPKAGSRGLT
SLADTFEHVIEELLDEDHKVRPNEENNKDADLYTSRVMLSSQVPLEPPLLFLLEEYKN
YLDAANMSMKRYGGFLRKYPKRSSEVAGEGDGDSMGHEDLYKRYGGFLRRIRPKL
KWDNQKRYGGFLRRQFKVVTRSQEDPNAYSGELFD
SEQ ID No. 43: pre-pro-Somatostatin - N-peptide 
MLSCRLQCALAALSIVLALGCVTGAPSDPRLRQFLQKSLAAAAGKQELAKYFLAELL
SEPNQTENDALEPEDLSQAAEQDEMRLELQR
SEQ ID No. 44: pre-pro-Somatostatin-pDyn Hybrid: 
MLSCRLQCALAALSIVLALGCVTGAPSDPRLRQFLQKSLAAAAGKQELAKYFLAELL
SEPNQTENDALEPEDLSQAAEQDEMRLELQRKRYGGFLRKYPKRSSEVAGEGDGDS
MGHEDLYKRYGGFLRRIRPKLKWDNQKRYGGFLRRQFKVVTRSQEDPNAYSGELFD
SEQ ID No. 45: pre-pro-Tachykinin A - N- peptide
   MKILVALAVFFLVSTQLFAEEIGANDDLNYWSDWYDSDQIKEELPEPFEHLLQRIA
SEQ ID No. 46: pre-pro-Tachykinin A-pDyn Hybrid: 
MKILVALAVFFLVSTQLFAEEIGANDDLNYWSDWYDSDQIKEELPEPFEHLLQRIAKR
YGGFLRKYPKRSSEVAGEGDGDSMGHEDLYKRYGGFLRRIRPKLKWDNQKRYGGF
LRRQFKVVTRSQEDPNAYSGELFD
SEQ ID No. 47: pre-pro-VIP - N-peptide 
MDTRNKAQLLVLLTLLSVLFSQTSAWPLYRAPSALRLGDRIPFEGANEPDQVSLKEDI
DMLQNALAENDTPYYDVSRNA
SEQ ID No. 48: pre-pro-VIP-pDyn Hybrid: 
MDTRNKAQLLVLLTLLSVLFSQTSAWPLYRAPSALRLGDRIPFEGANEPDQVSLKEDI
DMLQNALAENDTPYYDVSRNAKRYGGFLRKYPKRSSEVAGEGDGDSMGHEDLYKR
YGGFLRRIRPKLKWDNQKRYGGFLRRQFKVVTRSQEDPNAYSGELFD
SEQ ID No. 49: pre-pro CCK - N-peptide
   MNSGVCLCVLMAVLAAGALTQPVPPADPAGSGLQRAEEAPRRQL
SEQ ID No. 50: pre-pro CCK-pDyn Hybrid 
MNSGVCLCVLMAVLAAGALTQPVPPADPAGSGLQRAEEAPRRQLKRYGGFLRKYPK
RSSEVAGEGDGDSMGHEDLYKRYGGFLRRIRPKLKWDNQKRYGGFLRRQFKVVTRS
QEDPNAYSGELFD
SEQ ID No. 51: pre-pro Nociceptin - N-peptide 
MKVLLCDLLLLSLFSSVFSSCQRDCLTCQEKLHPALDSFDLEVCILECEEKVFPSPLWT
PCTKVMARSSWQLSPAAPEHVAAALYQPRASEMQHL
SEQ ID No. 52: pre-pro Nociceptin-pDyn Hybrid

MKVLLCDLLLLSLFSSVFSSCQRDCLTCQEKLHPALDSFDLEVCILECEEKVFPSPLWT
PCTKVMARSSWQLSPAAPEHVAAALYQPRASEMQHLKRYGGFLRKYPKRSSEVAGE
GDGDSMGHEDLYKRYGGFLRRIRPKLKWDNQKRYGGFLRRQFKVVTRSQEDPNAY
SGELFD

Subject matter of the present invention is a delivery vector comprising a DNA sequence encoding pre-prodynorphin or pre-prodynorphin-variants according to the present invention wherein the modified propeptide fragment is optionally flanked by peptidase recognition signals comprising K, R, KR, RK or RR.

Peptidase (prohormone convertase) recognition signals are known to a person skilled in the art and may be single or paired basic amino acids, preferably but not exclusively K, R, KR, RK or RR.

According to the above description a specific pre-prodynorphin with shortened modified propeptide fragment may be the following:

SEQ: 53
MAWQGLVLAACLLMFPSTTADCLSRCSLCAVKTQDGPKPINPLICSLQCQAALLPSEEWERC
QSFLSFFTPSTLGLNDKEDLGSKSVGEGPYSELAKLSGSFLRKEQVKRYGGFLRKYPKRSS
EVAGEGDGDSMGHEDLYKRYGGFLRRIRPKLKWDNQKRYGGFLRRQFKVVTRSQEDPNAY
SGELFDA.

The bold amino acids may represent amino acids of the sorting motif of POMC and were derived from analogy, the italic amino acids represent Dyn A, the underlined amino acids represent leumorphin and the bold and underlined amino acids represent neoendorphin. Lastly, the amino acids in bold and italic represent peptidase recognition signals.

According to the above description a specific pre-prodynorphin with hybrid modified propeptide fragment may be the following:

Hybrid pPOMC – ppDyn (SEQ ID No. 54):

MPRSCCSRSGALLLALLLQASMEVRGWCLESSQCQDLTTESNLLECIRACKPKEQVKRYGG

FLRKYPKRSSEVAGEGDGDSMGHEDLYKRYGGFLRRIRPKLKWDNQKRYGGFLRRQFKV

VTRSQEDPNAYSGELFDA

The bold amino acids represent amino acids of the sorting motif of POMC, the italic amino acids represent Dyn A, the underlined amino acids represent leumorphin and the bold and underlined amino acids represent neoendorphin. Lastly, the amino acids in bold and italic represent peptidase recognition signals and the non-peptide coding part of ppDyn was replaced by parts of pPOMC (shaded in grey).

Subject matter of the present invention is a delivery vector comprising a DNA sequence encoding pre-prodynorphin or pre-prodynorphin-variants, wherein the target cells are neuronal cells of the central nervous system.

An embodiment of the present invention is a delivery vector comprising a DNA sequence encoding pre-prodynorphin or pre-prodynorphin-variants, wherein the target cells are subtypes of principal neurons and GABAergic interneurons.

Subject matter of the present invention is a delivery vector comprising a DNA sequence encoding pre-prodynorphin or pre-prodynorphin-variants wherein the signal peptide is a short peptide sequence of from 10 to 30 amino acids at the N-terminal end of precursor-proteins that are destined into the lumen of the endoplasmatic reticulum.

An embodiment of the present invention is a delivery vector comprising a DNA sequence encoding pre-prodynorphin or pre-prodynorphin-variants wherein the signal peptide is a short peptide sequence of from 10 to 30 amino acids, at the N-terminal end of precursor-proteins that are destined into the lumen of the endoplasmatic reticulum, wherein a stretch of 5 to 16 amino acids tends to form a single alpha helix structure.

The core of the signal peptide contains a stretch of hydrophobic amino acids (about 5 to16 amino acids in length; that has a tendency to form a single alpha-helix and is also referred to as the "h-region". In addition, many signal peptides begin with a positively charged stretch of amino acids, which may help to enforce proper topology of the polypeptide during translocation by what is known as the positive-inside rule. Yet, the amino-acid sequence of signal peptides strongly varies even within the group of pre-proneuropeptides.

Signal peptides as described herein can exhibit a number of varying sequences. Non-limiting examples of signal peptides in general, and signal peptides under the present invention may be selected, but are not restricted to the group comprising:
MAWQGLVLAACLLMFPSTTA (SEQ ID No. 11)
MARFLTLCTWLLLLGPGLLATVRA (SEQ ID No. 12)
MLGNKRLGLSGLTLALSLLVCLGALAEA (SEQ ID No. 13)
MLSCRLQCALAALSIVLALGCVTG (SEQ ID No. 14)
MKILVALAVFFLVSTQLFA (SEQ ID No. 15)
MRIMLLFTAILAFSLA (SEQ ID No. 16)
MPRSCCSRSGALLLALLLQASMEVRG (SEQ ID No. 17)
MNSGVCLCVLMAVLAAGA (SEQ ID No. 18)
MKVLLCDLLLLSLFSSVFS (SEQ ID No. 19)
MQPTLLLSLLGAVGLAAVNS (SEQ ID No. 20)

For the avoidance of doubt according to the present invention sorting motif and signal peptide may be derived from the same pre-pro-neuropeptide or proteins sorted to large dense core vesicles, or from two different pre-pro-neuropeptides or proteins sorted to large dense core vesicles.

Subject matter of the present invention is a delivery vector, wherein said delivery vector leads to release-on-demand of dynorphins or dynorphin-variants with agonistic effects on human Kappa Opioid Receptors.

Subject matter of the present invention is a delivery vector wherein the variants have an amino acid sequence identity of at least 70 % within the first 8 AA from the N-terminal end of SEQ ID No. 2 (YGGFLRRI), SEQ ID No. 3 (YGGFLRRQ) or SEQ ID No. 4 (YGGFLRRQ), respectively.

Subject matter of the present invention is a delivery vector, wherein the variants have an amino acid sequence identity of at least 80 % within the first 8 AA from the N-terminal end of SEQ ID No. 2, SEQ ID No. 3 or SEQ ID No. 4, respectively.

Subject matter of the present invention is a delivery vector, wherein the variants have an amino acid sequence identity of at least 90 % within the first 8 AA from the N-terminal end of SEQ ID No. 2, SEQ ID No. 3 or SEQ ID No. 4, respectively.

In a specific embodiment subject of the invention is a delivery vector as above described, wherein said delivery vector comprises multiple DNA sequences encoding SEQ ID No. 2, SEQ ID No. 3 and/or SEQ ID No. 4 or variants thereof wherein the sequences according to SEQ ID No. 2, SEQ ID No. 3 and/or SEQ ID No. 4 or variants thereof are flanked by peptidase recognition signals.

This means as an example that said delivery vector may comprise a DNA sequence encoding SEQ ID No. 2 two times in a way that two molecules of a peptide according to SEQ ID No. 2 would be derived from one delivery vector.

Peptidase (prohormone convertase) recognition signals are known to a person skilled in the art and may be single or paired basic amino acids, preferably but not exclusively K, R, KR, RK or RR.

In a specific embodiment subject of the invention is a delivery vector as above described, wherein said delivery vector comprises multiple DNA sequences encoding SEQ ID No. 2 and/or SEQ ID No. 4 or variants thereof wherein the sequences according, SEQ ID No. 2 and/or SEQ ID No. 4 or variants thereof are flanked by peptidase recognition signals.

Subject matter of the present invention is a delivery vector according to the present invention, wherein said delivery vector comprises a DNA sequence encoding a pre-prodynorphin-variant that comprises at least one of the following sequences of variants selected from the group:
a. SEQ ID No. 21 (YGZFZ₁RRZRZKLKWDNQ)
b. SEQ ID No. 22 (YGZFZ₂RRZFZ₃VVT)
c. SEQ ID No. 23 (YGZFZ₄RRZFZ₅VVTRSQEDPNAYSGELFDA),
   wherein each instance of Z, as well as any of Z₁ to Z₅ stands each independently for any of the naturally occurring amino acids, and wherein at least one Z in a sequence according to a.; b. or c. is substituted by another amino acid when compared to the wild-type sequence of said dynorphin fragment according to a sequence according SEQ ID No 2, SEQ ID No. 3, SEQ ID No 4.
In one embodiment "Z₁ to Z₅" is selected from the group comprising alanine, glycine, asparagine, glutamine, leucine, histidine, methionine, serine, valine and isoleucine.

Subject matter of the present invention is a delivery vector according to the present invention, wherein said delivery vector comprises a DNA sequence encoding a pre-prodynorphin-variant that comprises at least one of the following sequences of variants selected from the group:
YGGFLRRQFKVVT (SEQ ID No. 3)
YGAFLRRQFKVVT (SEQ ID No. 24)
YGGFLRRAFKVVT (SEQ ID No. 25)
YGGFLRRQFAVVT (SEQ ID No. 26)
YGAFLRRAFKVVT (SEQ ID No. 27)
YGAFLRRQFAVVT (SEQ ID No. 28)
YGMFLRRQFKVVT (SEQ ID No. 29)
YGGFSRRQFKVVT (SEQ ID No. 30)
YGAFSRRQFKVVT (SEQ ID No. 31)
YGAFLRRHFKVVT (SEQ ID No. 32)
YGGFLRRHFKVVT (SEQ ID No. 33)
YGMFSRRQFKVVT (SEQ ID No. 34)
YGMFLRRHFKVVT (SEQ ID No. 35)

According to the present invention, the delivery vector drives expression of a pre-propeptide that is SEQ ID No. 3 (YGGFLRRQFKVVT), which is the wildtype sequence of Dyn B (rimorphin).

The variants of SEQ ID No. 3 (Dyn B) according to the present invention are characterized by substitutions of the amino acids on positions 3 (Glycine), 5 (Leucine), 8 (Glutamine) and 10 (Lysine) of Dyn B. These substitutions are preferably selected from the group comprising Alanine, Methionine, Serine and Histidine.

Subject matter of the present invention is a delivery vector wherein said delivery vector comprises in addition a recombinant adeno-associated virus (AAV) vector genome or a recombinant lentivirus genome.

The delivery vectors produced according to the present invention are useful for the delivery of nucleic acids to cells in vitro, ex vivo, and in vivo. In particular, the delivery vectors can be advantageously employed to deliver or transfer nucleic acids to animal, more preferably mammalian cells.

Suitable vectors include viral vectors (e.g., retrovirus, lentivirus, alphavirus; vaccinia virus; adenovirus, adeno-associated virus, or herpes simplex virus), lipid vectors, lipid nanoparticles, polylysine vectors, synthetic polyamino polymer vectors that are used with nucleic acid molecules, such as plasmids, and the like.

Any viral vector that is known in the art can be used in the present invention. Examples of such viral vectors include, but are not limited to vectors derived from: Adenoviridae; Adeno-associated Viridae (AAV), Birnaviridae; Bunyaviridae; Caliciviridae, Capillovirus group; Carlavirus group; Carmovirus virus group; Group Caulimovirus; Closterovirus Group; Commelina yellow mottle virus group; Comovirus virus group; Coronaviridae; PM2 phage group; Corcicoviridae; Group Cryptic virus; group Cryptovirus; Cucumovirus virus group Family ([PHgr]6 phage group; Cysioviridae; Group Carnation ringspot; Dianthovirus virus group; Group Broad bean wilt; Fabavirus virus group; Filoviridae; Flaviviridae; Furovirus group; Group Germinivirus; Group Giardiavirus; Hepadnaviridae; Herpesviridae; Hordeivirus virus group; Illarvirus virus group; Inoviridae; Iridoviridae; Leviviridae; Lipothrixviridae; Luteovirus group; Marafivirus virus group; Maize chlorotic dwarf virus group; icroviridae; Myoviridae; Necrovirus group; Nepovirus virus group; Nodaviridae; Orthomyxoviridae; Papovaviridae; Paramyxoviridae; Parsnip yellow fleck virus group; Partitiviridae; Parvoviridae; Pea enation mosaic virus group; Phycodnaviridae; Picomaviridae; Plasmaviridae; Prodoviridae; Polydnaviridae; Potexvirus group; Potyvirus; Poxviridae; Reoviridae; Retroviridae; Rhabdoviridae; Group Rhizidiovirus; Siphoviridae; Sobemovirus group; SSV 1-Type Phages; Tectiviridae; Tenuivirus; Tetraviridae; Group Tobamovirus; Group Tobravirus; Togaviridae; Group Tombusvirus; Group Tobovirus; Totiviridae; Group Tymovirus; and Plant virus satellites. Protocols for producing recombinant viral vectors and for using viral vectors for nucleic acid delivery can be found in (Ausubel et al., 1989) and other standard laboratory manuals (e.g., Rosenzweig et al. 2007). Particular examples of viral vectors are those previously employed for the delivery of nucleic acids including, for example, retrovirus, lentivirus, adenovirus, adeno-associated virus (AAV) and other parvoviruses, herpes virus, and poxvirus vectors. The term "parvovirus" as used herein encompasses the family Parvoviridae, including autonomous parvoviruses, densoviruses and dependoviruses. The term adeno-associated virus (AAV) includes all vertebrate variants especially of human, primate, other mammalian, avian or serpentine origin. The autonomous parvoviruses include members of the genera Parvovirus, Erythrovirus, Bocavirus, Densovirus, Iteravirus, and Contravirus. Exemplary autonomous parvoviruses include, but are not limited to, minute virus of mice, bovine parvovirus, canine parvovirus, chicken parvovirus, feline panleukopenia virus, feline parvovirus, goose parvovirus, HI parvovirus, muscovy duck parvovirus, bocavirus, bufavirus, tusavirus and B19 virus, and any other virus classified by the International Committee on Taxonomy of Viruses (ICTV) as a parvovirus. Other autonomous parvoviruses are known to those skilled in the art. See, e.g. (Berns et al. 2013).

In one embodiment of the invention said delivery vector comprises in addition a recombinant adeno-associated virus (AAV) vector genome or a recombinant lentivirus genome.

In one particular embodiment of the invention said delivery vector comprises in addition a recombinant AAV vector, wherein preferably said vector is a serotype of human or primate origin.

Subject matter of the present invention is a delivery vector comprising a recombinant adeno-associated virus (AAV) vector genome comprising inverted terminal repeats (ITR) preferably derived from AAV serotype 2, alternatively from AAV serotype 1, 3, 4, 5, 6, 7, 8, 9, 10, rh10, 11, 12, 13, 14, or reengineered variants thereof, wherein said vector genome is packaged in an AAV capsid selected from the group comprising AAV serotypes 1, 2, 3, 4, 5, 6, 7, 8, 9, 10, rh10, 11, 12, 13, 14, serpentine AAV, ancestral AAV, AAV-TT, AAVv66; AAV1P4, AAV1P5, AAV-PHP.B, AAV-PHP.eB, AAV2-HBKO, AAV.CAP-B10, AAV.CAP-MAC, AAV2.NN or further AAV capsid mutants derived thereof, particularly of AAV serotype 1 or 2, further particularly AAV1 and capsids derived thereof including AAV1P4, AAV1P5 or AAV2 and capsids derived thereof including AAV2-NN (Börner et al. MolTher 2020; Pavlou et al EMBOMolMed 2021; Challis AnnRev Neuroscience 2022; Naidoo et al MolTher 2018; Hsu et al NatureComm 2020; Tordo Brain 2018) .

In one particular embodiment of the invention said delivery vector is a single-stranded (ssAAV) vector or a self-complimentary vector (scAAV) also referred to as dimeric or duplex AAV vector (McCarty et al. Gene Ther 2001).

In one particular embodiment of the invention said delivery vector is a delivery vector as described above, wherein the DNA sequence encoding pre-prodynorphyin or pre-prodynorphin-variants is operatively linked to expression control elements comprising a promoter and/or enhancer that induce sufficient expression of the gene product of interest to obtain a therapeutic effect.

For example, the encoding nucleic acid may be operably associated with expression control elements, such as promoters, enhancers, other transcription / translation control signals, origins of replication, polyadenylation signals, and/or internal ribosome entry sites (IRES) and the like. It will further be appreciated that a variety of promoter / enhancer elements may be used depending on the level and tissue-specific expression desired. The promoter / enhancer may be constitutive or inducible, depending on the pattern of expression desired. The promoter / enhancer may be native or foreign and can be a natural or a synthetic sequence. By foreign, it is intended that the transcriptional initiation region is not found in the wild-type host into which the transcriptional initiation region is introduced. Promoter / enhancer elements that are functional in the target cell or subject to be treated are most preferred. Mammalian promoter / enhancer elements are also preferred. Most preferred are promoter / enhancer elements active in human neurons and not, or to a lesser extend in glial cells. The promoter / enhancer element may express the transgene constitutively or inducibly.

Exemplary constitutive promoters include, but are not limited to a Beta-actin promoter, a cytomegalovirus promoter, a cytomegalovirus-enhancer/chicken beta-actin hybrid promoter, and a Rous sarcoma virus promoter. Inducible expression control elements are generally employed in those applications in which it is desirable to provide regulation over expression of the heterologous nucleic acid sequence(s). Inducible promoters / enhancer elements for gene delivery include neuron-specific, brain-specific, muscle specific (including cardiac, skeletal and / or smooth muscle), liver specific, bone marrow specific, pancreatic specific, spleen specific, and lung specific promoter/enhancer elements. In particular embodiments, the promoter/enhancer is functional in cells or tissue of the CNS and may even be specific to cells or tissues of the CNS. Such promoters / enhancers include but are not limited to promoters/enhancers that function in the eye (e.g., retina and cornea), neurons (e.g., the neuron specific enolase, AADC, human synapsin (hSYN), phosphoglycerate kinase (PGK), or serotonin receptor promoter), glial cells (e.g., S100 or glutamine synthase promoter), and oligodendrocytes. Other promoters that have been demonstrated to induce transcription in the CNS include, but are not limited to, myelin basic protein (MBP) promoter (Tani et al., 1996), and the prion promoter (Loftus et al., 2002). Preferred is a neuron-specific promoter displaying significantly reduced, preferably no expression in glial cells.

Other inducible promoter / enhancer elements include drug-inducible, hormone-inducible and metal-inducible elements, and other promoters regulated by exogenously supplied compounds, including without limitation, the zinc-inducible metallothionein (MT) promoter; the dexamethasone (Dex)- inducible mouse mammary tumor virus (MMTV) promoter; the T7 polymerase promoter system (see WO 98/10088); the ecdysone-inducible insect promoter (No et al, 1996); the tetracycline-repressible system (Gossen and Bujard, 1992); the tetracycline-inducible system (Gossen et al., 1995); see also (Harvey et al., 1998); the RU486-inducible system (Wang, DeMayo et al., 1997); (Wang, Xu et al., 1997); and the rapamycin-inducible system (Magari et al., 1997).

In a particular embodiment of the invention the promoter and/or enhancer is selected from the group comprising constitutively active promoters e.g. CMV (cytomegalovirus immediate-early gene enhancer/promoter)- or CBA promoter (chicken beta actin promoter and human cytomegalovirus IE gene enhancer), or inducible promoters comprising Gene Switch, tet-operon derived promotor, or neuron-specific promoters derived of e.g. phosphoglycerate kinase (PGK), synapsin-1 (SYN), neuron-specific enolase (NSE), preferably but not exclusively of human origin.

In a particular embodiment of the invention said delivery vector further comprises a posttranscriptional regulatory element, preferably the woodchuck-hepatitis-virus-posttranscriptional-regulatory element (WPRE) or shortened variants derived thereof (Loeb et al. HumGeneTher 1999). Other possible posttranscriptional regulatory elements are known to a person skilled in the art.

Subject matter of the present invention is a recombinant virus particle or a liposome or a nanoparticle comprising a delivery vector according to the invention.

Subject matter of the present invention is the recombinant virus particle or liposome, or nanoparticle wherein said delivery vector comprises in addition a recombinant adeno-associated virus (AAV) vector genome and said rAAV vector genome is encapsidated in an AAV capsid or wherein said delivery vector comprises in addition a recombinant lentivirus vector genome and is packaged in a lentivirus particle.

Subject of the present invention is furthermore a recombinant gene therapy vector comprising the foreign, therapeutic coding sequence, which is flanked by genetic elements for its expression and by virus-specific *cis* elements for its replication, genome packaging, genomic integration etc. The said virus genome is encapsidated as virus particle consisting of virus-specific proteins as in the case of AAV. In the case of lentivirus vectors the viral genome and virus-specific proteins, like reverse transcriptase and others are encapsidated into lentivirus capsids. These are enveloped by a lipid bilayer into which virus-specific proteins are embedded. Liposomes comprise the above-described nucleotide sequences or entire DNA backbones including all regulatory elements of the gene therapy-, or delivery vector.

Examples of liposomes include DSPC (1,2-distearoyl-sn-glycero-3-phosphocholine, cholesterol, DSPE-PEG2000 (1,2-distearoyl-sn-glycero-3-phosphoethanol- amine-N-[amino(polyethylene glycol)-2000], or DSPE- PEG2000-mal (1,2-distearoyl-sn-glycero-3-phosphoethanol- amine-N-[maleimide(polyethylene glycol)-2000] or variants comprising sphingomyelin / cholesterol and phosphatidic acid.

In one particular embodiment of the invention said delivery vector comprises in addition a recombinant adeno-associated virus (AAV) vector genome and said recombinant AAV (rAAV) vector genome is encapsidated in an AAV capsid.

Adeno-associated viruses (AAV) have been developed as nucleic acid delivery vectors. For a review, see (Muzyczka, 1992) (Li and Samulski, Nature Rev Genetics 2020). AAV are helper-dependent parvoviruses requiring a helper virus, typically adenovirus or herpesvirus for productive replication. AAV represent a growing family of at least 14 naturally occurring serotypes of human or primate origin. AAVs of other mammalian species, or of avian or insect origin have been described (see Berns et al., 2013). The AAVs have small icosahedral capsids, 18-26 nanometers in diameter and contain a single-stranded DNA genome of 4 - 5 kilobases in length. AAV encapsidates both AAV DNA strands, either the sense or antisense DNA strand is incorporated into one virion. The AAV genome carries two major open reading frames encoding the genes *rep* and *cap.* Rep encodes a family of overlapping, nonstructural, regulatory proteins. In the best-studied AAV prototype strain, AAV2, the mRNAs for Rep78 and Rep68 are transcribed from the AAV p5 promoter (Stutika et al. 2015). Rep78/68 are required for AAV transcription, AAV DNA replication, AAV integration into the host cell genome and its rescue therefrom. Rep52 and Rep40 represent N-terminally truncated versions of Rep78 and Rep68 transcribed from a separate promoter, p19 and are required for encapsidation of the newly synthesized AAV genome into preformed AAV capsids. These are formed by the three *cap* gene-derived proteins, VP1, VP2, and VP3. The *cap* ORF also encodes AAP, an assembly-enhancing protein, and an AAV egress-promoting factor called MAAP. AAP and MAAP do not form part of the capsid (Sonntag et al. PNAS 2010; Elmore et al. NatureCom 2021). The AAV ORFs are flanked by inverted terminal repeat sequences (ITRs) at either end of the genome. These vary in length between AAV serotypes, in AAV2 these comprise around 145 bp, the first 125 bp thereof are capable of forming Y- or T-shaped duplex structures. The ITRs comprise terminal resolution sites (trs) where the replicated concatemeric AAV genome is nicked by Rep to form unit length ssAAV genomes ready for packaging into AAV capsids. The ITRs represent the minimal AAV sequences required *in cis* for DNA replication, packaging, genomic integration and rescue. Only these have to be retained in an AAV vector to ensure DNA replication and packaging of the AAV vector genome. Foreign genes flanked by AAV-ITRs will be replicated and packaged into AAV capsids provided the AAV genes *rep* and *cap* are expressed *in trans* in the chosen packaging cell (Muzyczka, 1992). In the case of scAAV the terminal resolution site (trs) is deleted in one of the ITRs, so that the AAV genome cannot be nicked by Rep on the affected end, thereby being retained as unresolved duplex, self-complementary (sc)AAV genome.

AAV are among the few viruses that can persist over months and years in non-dividing cells *in vivo*, including neurons, muscle, liver, heart and others. Wildtype AAV2 has been shown to integrate its genome into the host cell genome in a Rep78/68-dependent manner, with a preference for chromosomal loci with DNA sequence homology to the so-called Rep-binding site which forms part of the AAV-ITRs (Hüser et al., 2014). In contrast, AAV vectors mostly persist as concatemeric nuclear episomes. Devoid of the AAV genes *rep* and *cap* AAV vectors rarely integrate at all, and if so without genomic preference (Hüser et al., 2014). Nonetheless long term AAV persistence has been shown in non-dividing, postmitotic cells including neurons which renders AAV vectors ideal for CNS transduction and long-term gene addition therapy of chronic diseases of genetic or acquired origin.

Generally, a recombinant AAV vector (rAAV) genome will only retain the inverted terminal repeat (ITR) sequence(s) in its native (ssAAV) or trs-deleted (scAAV) version, so as to maximize the size of the transgene that can be efficiently packaged by the vector. The structural- and non-structural protein-coding sequences may be provided *in trans*, e.g., from a vector, such as a plasmid, by stably integrating the respective genes into a packaging cell, or in a recombinant helper virus such as HSV or baculovirus, as reviewed in (Mietzsch, Grasse et al., 2014). Typically, the rAAV vector genome comprises at least one AAV inverted terminal repeat (ITR), more typically two AAV inverted terminal repeats, which will generally be at the 5' and 3' ends of the heterologous nucleotide sequence(s). The AAV ITR may be from any AAV including serotypes 1-14. Since AAV2-derived ITRs can be cross-packaged into virtually any AAV serotype capsids, AAV2 ITRs combined with AAV2 *rep* are mostly employed. The AAV terminal repeats need not maintain the wild-type terminal repeat sequence (e.g., a wild-type sequence may be altered by insertion, deletion, truncation or missense mutations), as long as the terminal repeat mediates the desired functions, e.g., DNA replication, virus packaging, integration, and/or provirus rescue, and the like. The rAAV vector genome spans generally about 70% to about 105% of the size of the wild-type genome and comprises an appropriate packaging signal as part of the AAV-ITR. To facilitate packaging into an AAV capsid, the entire vector genome (from ITR to ITR) is preferably below 5.2 kb, more preferably up to 4.8kb in size to allow packaging of the entire recombinant genome into the preformed AAV capsid. So-called dimeric or self-complementary AAV vectors (scAAV) were developed to package double-stranded instead of single-stranded AAV genomes (McCarty et al., 2001). scAAVs lead to enhanced AAV gene expression, however at the price of reduced transgene capacity. The total packaging capacity is only 2.4kb (from ITR to ITR), which is enough for small genes or cDNAs including those for neuropeptides.

Any suitable method known in the art can be used to produce AAV vectors expressing the nucleic acids of this invention. AAV vector stocks can be produced by co-transfection of plasmids for the ITR-flanked AAV vector genome expressing the transgene together with an AAV rep/cap expressing plasmid of the desired serotype and adenovirus-derived helper genes for AAV replication (Grimm et al., 2003; Xiao et al., 1998). AAV vectors can also be produced in packaging cell lines of mammalian or insect origin and/or in combination with recombinant helper viruses, such as adenovirus, herpes simplex virus (HSV), another member of the herpesvirus family, or baculovirus, as reviewed and discussed in (Mietzsch, Grasse et al., 2014).

Subject matter of the present invention is a delivery vector comprising a DNA sequence encoding pre-prodynorphin or pre-prodynorphin-variants as detailed herein.

Subject matter of the present invention is a delivery vector or recombinant virus particle or liposome or nanoparticle for use in delivering a nucleic acid to a cell of the central nervous system, comprising contacting the cell with the delivery vector or recombinant virus particle or liposome or nanoparticle under conditions sufficient for the DNA sequence encoding pre-prodynorphin or pre-prodynorphin-variants to be introduced into the cell, more specifically into the cell nucleus.

The delivery vectors of the present invention provide a means for delivering nucleic acid sequences into cells of the central nervous system, preferably neurons. The delivery vectors may be employed to transfer a nucleotide sequence of interest to a cell in vitro, e.g., to produce a polypeptide in vitro or for ex vivo gene therapy. The vectors are additionally useful in a method of delivering a nucleotide sequence to a subject in need thereof. In this manner, the polypeptide may thus be produced in vivo in the subject. The subject may be in need of the polypeptide because the subject has a deficiency of the polypeptide, or because the production of the polypeptide in the subject may impart some therapeutic effect, as a method of treatment or otherwise, and as explained further below.

In one particular embodiment of the method of delivering a nucleic acid to a cell of the central nervous system the pre-prodynorphin or pre-prodynorphin-variant is produced processed and mature dynorphin peptides or variants thereof released from the cell.

In one particular embodiment of the method of delivering a nucleic acid to a cell of the central nervous system the method comprises contacting the cell with the recombinant virus particle or liposome or nanoparticle as described above under conditions sufficient for the DNA sequence encoding pre-prodynorphin or pre-prodynorphin-variants to be introduced into the cell nucleus. Conditions sufficient for the DNA sequence encoding pre-prodynorphin or pre-prodynorphin-variants to be introduced into the cell is the contacting of the AAV capsid to host cell surface receptors and coreceptors. AAV1 capsids bind to 2-3 sialic acid linked to *N-*acetylgalactosamine, followed by 1-4-linked *N*-acetylglucosamine, whereas AAV2 capsids bind to heparin sulfate proteoglycan particularly 6-O- and N-sulfated heparins on the cell surface (Mietzsch, Broecker et al., 2014). AAV coreceptors include FGFR-1, Integrin aVb5, hepatocyte growth factor receptor (c-met) and the universal AAV receptor, AAVR necessary for transduction with AAV1, AAV2 and other serotypes irrespective of the presence of specific glycans (Pillay et al., 2016). AAVR directly binds to AAV particles and helps trafficking to the trans Golgi network. AAV2 has been described to use the nuclear pore complex for nuclear entry thereby interacting with importin-β alone or in complex with other import proteins (Nicolson and Samulski JVirol 2014). Most parvoviruses and AAV serotypes use similar mechanisms for nuclear entry (Mattola et al. MolMicrobiol _2022), AAV vectors are assembled in the cell nucleus .

Subject matter of the present invention is a delivery vector or recombinant virus particle or liposome or nanoparticle for use as medicament.

Subject matter of the present invention is a delivery vector or recombinant virus particle or liposome or nanoparticle as detailed herein for use in treating focal epilepsy in a subject, in particular mesial temporal lobe epilepsy, or for use in preventing epileptic seizures in a subject that suffers from focal epilepsy through activation of human Kappa Opioid Receptors in the epileptogenic focus, thereby inhibiting seizures.

Particularly, the delivery vector or recombinant virus particle or liposome or nanoparticle as detailed herein is able to deliver a DNA sequence encoding pre-prodynorphin or pre-prodynorphin-variants as described herein, which in turn drives expression of a pre-propeptide in a target cell, enabling the release of dynorphin or dynorphin-variants from the target cell on demand as described herein, thereby leading to activation of human Kappa Opioid Receptors in the epileptogenic focus.

Subject matter of the present invention is a delivery vector or recombinant virus particle or liposome or nanoparticle for use in treating focal epilepsy in a subject, in particular mesial temporal lobe epilepsy, or for use in preventing epileptic seizures in a subject that suffers from focal epilepsy through activation of human Kappa Opioid Receptors in the epileptogenic focus, thereby inhibiting seizures and/or through to on-demand release of peptides with agonistic effects on human Kappa Opioid Receptors in the epileptogenic focus.

Subject matter of the present invention is a delivery vector or recombinant virus particle or a liposome or nanoparticle for use in treating focal epilepsy in a subject, in particular mesial temporal lobe epilepsy, or for use in preventing epileptic seizures in a subject that suffers from focal epilepsy wherein said vector or recombinant virus particle or liposome or nanoparticle is suitable for peripheral administration or for intracranial or for intracerebral or for intrathecal or for intraparenchymal administration.

Subject matter of the present invention is a delivery vector or recombinant virus particle or a liposome or nanoparticle for use in treating focal epilepsy in a subject, in particular mesial temporal lobe epilepsy, or for use in preventing epileptic seizures in a subject that suffers from focal epilepsy, wherein said delivery vector or recombinant virus particle or a liposome or nanoparticle is applied intracerebral, preferred is applied focal.

Subject matter of the present invention is a pharmaceutical release-on-demand composition, delivery vector or recombinant virus particle or liposome or nanoparticle, and optionally a pharmaceutically acceptable carrier.

Subject matter of the present invention is a cell infected, preferably in vitro or ex vivo, with a delivery vector or recombinant virus or liposome or nanoparticle.

Subject matter of the present invention is a method of treating a subject with focal epilepsy in particular mesial temporal lobe epilepsy, or a method of preventing epileptic seizures in a subject that suffers from focal epilepsy comprising administering a delivery vector, a recombinant virus particle or a liposome or nanoparticle, or a pharmaceutical composition to the subject, whereby preferably said delivery vector or recombinant virus particle or liposome or nanoparticle encode pre-propeptides, which after maturation and release provide activation of human Kappa Opioid Receptors in the epileptogenic focus, thereby inhibiting seizures, and wherein preferably said delivery vector or recombinant virus particle or a liposome or nanoparticle is applied intracerebral, intraparenchymal, preferably applied focal.

Subject matter of the present invention is a peptide with agonistic effects on human Kappa Opioid Receptors (KOR) wherein said peptide is selected from the group comprising the peptides:
SEQ ID No. 21 (YGZFZ₁RRZRZKLKWDNQ)
SEQ ID No. 22 (YGZFZ₂RRZFZ₃VVT)
SEQ Id No. 23 (YGZFZ₄RRZFZ₅VVTRSQEDPNAYSGELFDA

Subject matter of the present invention is a peptide with agonistic effects on human Kappa Opioid Receptors (KOR) wherein said peptide is selected from the group comprising the peptides:
YGAFLRRQFKVVT (SEQ ID No. 24)
YGGFLRRAFKVVT (SEQ ID No. 25)
YGGFLRRQFAVVT (SEQ ID No. 26)
YGAFLRRAFKVVT (SEQ ID No. 27)
YGAFLRRQFAVVT (SEQ ID No. 28)
YGMFLRRQFKVVT (SEQ ID No. 29)
YGGFSRRQFKVVT (SEQ ID No. 30)
YGAFSRRQFKVVT (SEQ ID No. 31)
YGAFLRRHFKVVT (SEQ ID No. 32)
YGGFLRRHFKVVT (SEQ ID No. 33)
YGMFSRRQFKVVT (SEQ ID No. 34)
YGMFLRRHFKVVT (SEQ ID No. 35)

### Description of the below sequences:

**SEQ ID No. 1 (ppDyn)** Human pre-prodynorphin before processing as expressed in the human brain.
**SEQ ID No. 2 Dyn A**
   YGGFLRRIRPKLKWDNQ
**SEQ ID No. 3 Dyn B (rimorphin)**
   YGGFLRRQFKVVT
**SEQ ID No. 4: Leumorphin**
   YGGFLRRQFKVVTRSQEDPNAYSGELFDA
**SEQ ID No. 5: Unmodified propeptide fragment of ppDyn** 
DCLSRCSLCA VKTQDGPKPI NPLICSLQCQ AALLPSEEWE RCQSFLSFFT
PSTLGLNDKE DLGSKSVGEG PYSELAKLSG SFLKELEKSK FLPSISTKEN
TLSKSLEEKL RGLSDGFREG AESELMRDAQ LNDGAMETGT LYLAEEDPKE QV
**SEQ ID No. 6: Modified propeptide fragment of ppDyn** 
DCLSRCSLCA VKTQDGPKPI NPLICSLQCQ AALLPSEEWE RCQSFLSFFT
PSTLGLNDKE DLGSKSVGEG PYSELAKLSG SFLRKEQVKR
**SEQ ID No. 7 Modified propeptide fragment of ppDyn** 
DLGSKSVGEG PYSELAKLSG SFLKELEKSK FLPSISTKEN TLSKSLEEKL
RGLSDGFREG AESELMRDAQ LNDGAMETGT LYLAEEDPKE QV
**SEQ ID No. 8 Modified propeptide fragment of ppDyn**
   DLGSKSVGEG PYSELAKLSG SFLRKE QV
**SEQ ID No. 9 Modified propeptide fragment of ppDyn**
   DLGSKSVGEG PYSELAKLRKE QV
**SEQ ID No. 10: N-terminal part of pPOMC**
   MPRSCCSRSGALLLALLLQASMEVRGWCLESSQCQDLTTESNLLECIRACKP
**SEQ ID No. 11: Signal peptide of ppdynorphin**
   MAWQGLVLAACLLMFPSTTA
**SEQ ID No. 12: Signal peptide of ppenkephalin**
   MARFLTLCTWLLLLGPGLLATVRA
**SEQ ID No. 13: Signal peptide of ppneuropeptide Y**
   MLGNKRLGLSGLTLALSLLVCLGALAEA
**SEQ ID No. 14: Signal peptide of ppsomatostatin**
   MLSCRLQCALAALSIVLALGCVTG
**SEQ ID No. 15: Signal peptide of pptachykinin A**
   MKILVALAVFFLVSTQLFA
**SEQ ID No. 16: Signal peptide of pptachykinin B**
   MRIMLLFTAILAFSLA
**SEQ ID No. 17: Signal peptide of pproopiomelanocortin**
   MPRSCCSRSGALLLALLLQASMEVRG
**SEQ ID No. 18: Signal peptide of ppcholecystokinin**
   MNSGVCLCVLMAVLAAGA
**SEQ ID No. 19: Signal peptide of ppnociceptin**
   MKVLLCDLLLLSLFSSVFS
**SEQ ID No. 20: Signal peptide of ppchromogranin B**
   MQPTLLLSLLGAVGLAAVNS
**SEQ ID No. 21 Dyn A modified**
   YG**Z**F**Z₁**RR**Z**R**Z**KLKWDNQ
   Each Z and Z₁ each independently stands for any amino acid, at least one is preferably substituted by another amino acid in comparison to the wild-type sequence.
**SEQ ID No. 22 Dyn B modified**
   YG**Z**F**Z₂**RR**Z**F**Z₃**VVT
   Each Z, Z₂ and Z₃ each independently stands for any amino acid, at least one is preferably substituted by another amino acid in comparison to the wild-type sequence.
**SEQ ID No. 23: Leumorphin modified**
   YG**Z**F**Z₄**RR**Z**F**Z₅**VVTRSQEDPNAYSGELFDA
   Each Z, Z₄ and Z₅ each independently stands for any amino acid, at least one is preferably substituted by another amino acid in comparison to the wild-type sequence which is SEQ ID No. 4.

### Dyn B Modified:

**SEQ ID No. 24**
   YGAFLRRQFKVVT
**SEQ ID No. 25**
   YGGFLRRAFKVVT
**SEQ ID No. 26**
   YGGFLRRQFAVVT
**SEQ ID No. 27**
   YGAFLRRAFKVVT
**SEQ ID No. 28**
   YGAFLRRQFAVVT
**SEQ ID No. 29**
   YGMFLRRQFKVVT
**SEQ ID No. 30**
   YGGFSRRQFKVVT
**SEQ ID No. 31**
   YGAFSRRQFKVVT
**SEQ ID No. 32**
   YGAFLRRHFKVVT
**SEQ ID No. 33**
   YGGFLRRHFKVVT
**SEQ ID No. 34**
   YGMFSRRQFKVVT
**SEQ ID No. 35**
   YGMFLRRHFKVVT
**Sorting Motif: SEQ ID No. 36**
   DLXₓEX_{y}L
   wherein x is an integer from 1 to 20, y is an integer from 1 to 10, and each instance of X may independently be any amino acid.
**SEQ ID No. 37: pre-proenkephalin - N-peptide** 
MARFLTLCTWLLLLGPGLLATVRAECSQDCATCSYRLVRPADINFLACVMECEGKLP
SLKIWETCKELLQLSKPELPQDGTSTLRENSKPEESHLLA
**SEQ ID No. 38: pre-proenkephalin-pDyn Hybrid** 
MARFLTLCTWLLLLGPGLLATVRAECSQDCATCSYRLVRPADINFLACVMECEGKLP
SLKIWETCKELLQLSKPELPQDGTSTLRENSKPEESHLLAKRYGGFLRKYPKRSSEVA
GEGDGDSMGHEDLYKRYGGFLRRIRPKLKWDNQKRYGGFLRRQFKVVTRSQEDPN
AYSGELFDA
**SEQ ID No. 39: pre-pro-NPY - N-peptide**
   MLGNKRLGLSGLTLALSLLVCLGALAEA
**SEQ ID No. 40: pre-pro-NPY-pDyn Hybrid** 
MLGNKRLGLSGLTLALSLLVCLGALAEAKRYGGFLRKYPKRSSEVAGEGDGDSMGH
EDLYKRYGGFLRRIRPKLKWDNQKRYGGFLRRQFKVVTRSQEDPNAYSGELFD
**SEQ ID No. 41: pre-pro BDNF - N-peptide** 
MTILFLTMVISYFGCMKAAPMKEANIRGQGGLAYPGVRTHGTLESVNGPKAGSRGLT
SLADTFEHVIEELLDEDHKVRPNEENNKDADLYTSRVMLSSQVPLEPPLLFLLEEYKN
YLDAANMSM
**SEQ ID No. 42: pre-pro BDNF-pDyn Hybrid** 
MTILFLTMVISYFGCMKAAPMKEANIRGQGGLAYPGVRTHGTLESVNGPKAGSRGLT
SLADTFEHVIEELLDEDHKVRPNEENNKDADLYTSRVMLSSQVPLEPPLLFLLEEYKN
YLDAANMSMKRYGGFLRKYPKRSSEVAGEGDGDSMGHEDLYKRYGGFLRRIRPKL
KWDNQKRYGGFLRRQFKVVTRSQEDPNAYSGELFD
**SEQ ID No. 43: pre-pro-Somatostatin - N-peptide** 
MLSCRLQCALAALSIVLALGCVTGAPSDPRLRQFLQKSLAAAAGKQELAKYFLAELL
SEPNQTENDALEPEDLSQAAEQDEMRLELQR
**SEQ ID No. 44: pre-pro-Somatostatin-pDyn Hybrid** 
MLSCRLQCALAALSIVLALGCVTGAPSDPRLRQFLQKSLAAAAGKQELAKYFLAELL
SEPNQTENDALEPEDLSQAAEQDEMRLELQRKRYGGFLRKYPKRSSEVAGEGDGDS
MGHEDLYKRYGGFLRRIRPKLKWDNQKRYGGFLRRQFKVVTRSQEDPNAYSGELFD
**SEQ ID No. 45: pre-pro-Tachykinin A - N- peptide**
   MKILVALAVFFLVSTQLFAEEIGANDDLNYWSDWYDSDQIKEELPEPFEHLLQRIA
**SEQ ID No. 46: pre-pro-Tachykinin A-pDyn Hybrid** 
MKILVALAVFFLVSTQLFAEEIGANDDLNYWSDWYDSDQIKEELPEPFEHLLQRIAKR
YGGFLRKYPKRSSEVAGEGDGDSMGHEDLYKRYGGFLRRIRPKLKWDNQKRYGGF
LRRQFKVVTRSQEDPNAYSGELFD
**SEQ ID No. 47: pre-pro-VIP - N-peptide** 
MDTRNKAQLLVLLTLLSVLFSQTSAWPLYRAPSALRLGDRIPFEGANEPDQVSLKEDI
DMLQNALAENDTPYYDVSRNA
**SEQ ID No. 48: pre-pro-VIP-pDyn Hybrid** 
MDTRNKAQLLVLLTLLSVLFSQTSAWPLYRAPSALRLGDRIPFEGANEPDQVSLKEDI
DMLQNALAENDTPYYDVSRNAKRYGGFLRKYPKRSSEVAGEGDGDSMGHEDLYKR
YGGFLRRIRPKLKWDNQKRYGGFLRRQFKVVTRSQEDPNAYSGELFD
**SEQ ID No. 49: pre-pro CCK - N-peptide**
   MNSGVCLCVLMAVLAAGALTQPVPPADPAGSGLQRAEEAPRRQL
SEQ ID No. 50: pre-pro CCK-pDyn Hybrid 
MNSGVCLCVLMAVLAAGALTQPVPPADPAGSGLQRAEEAPRRQLKRYGGFLRKYPK
RSSEVAGEGDGDSMGHEDLYKRYGGFLRRIRPKLKWDNQKRYGGFLRRQFKVVTRS
QEDPNAYSGELFD
SEQ ID No. 51: pre-pro Nociceptin - N-peptide 
MKVLLCDLLLLSLFSSVFSSCQRDCLTCQEKLHPALDSFDLEVCILECEEKVFPSPLWT
PCTKVMARSSWQLSPAAPEHVAAALYQPRASEMQHL
**SEQ ID No. 52: pre-pro Nociceptin-pDyn Hybrid** 
MKVLLCDLLLLSLFSSVFSSCQRDCLTCQEKLHPALDSFDLEVCILECEEKVFPSPLWT
PCTKVMARSSWQLSPAAPEHVAAALYQPRASEMQHLKRYGGFLRKYPKRSSEVAGE
GDGDSMGHEDLYKRYGGFLRRIRPKLKWDNQKRYGGFLRRQFKVVTRSQEDPNAY
SGELFD
**Shortened modified ppDyn SEQ ID No. 53** 
MAWQGLVLAACLLMFPSTTADCLSRCSLCAVKTQDGPKPINPLICSLQCQAALLPSEE
WERCQSFLSFFTPSTLGLNDKEDLGSKSVGEGPYSELAKLSGSFLRKEQVKRYGGFL
RKYPKRSSEVAGEGDGDSMGHEDLYKRYGGFLRRIRPKLKWDNQKRYGGFLRRQFK
VVTRSQEDPNAYSGELFDA.
**Hybrid pPOMC - ppDyn: SEQ ID No 54** 
MPRSCCSRSGALLLALLLQASMEVRGWCLESSQCQDLTTESNLLECIRACKPKEQVK
RYGGFLRKYPKRSSEVAGEGDGDSMGHEDLYKRYGGFLRRIRPKLKWDNQKRYGGF
LRRQFKVVTRSQEDPNAYSGELFDA
 **Modified propeptide fragments of ppDyn**
**SEQ ID No. 55**
   **DL**GSKSVGEG PYS**EL RK**E QV.
**SEQ ID No.: 56**
   **DL**GSKSVGEG PYS**E**LAK**L**SG SFLK KE QV
**SEQ ID No.: 57**
   **DL**GSKSVG**E**G PYS**EL** AK**L**
**SEQ ID No.: 58**
   **DL**GSKSVG**E**G PYSE**L**

Particular embodiments of the present invention are
1. A delivery vector comprising a DNA sequence encoding pre-prodynorphin or pre-prodynorphin-variants and
   - wherein said delivery vector drives expression of a pre-propeptide in a target cell, and
   - wherein said delivery vector comprising said DNA sequence enables the release of dynorphin or dynorphin-variants from the target cell on demand, and
   - wherein said pre-propeptide is pre-prodynorphin or a pre-prodynorphin-variant and
   - wherein said pre-propeptide comprises a signal peptide, wherein the signal peptide is a N-terminal extension of a nascent polypeptide chain and wherein said signal peptide mediates protein targeting to the lumen of the endoplasmatic reticulum, and,
   - wherein said pre-propeptide comprises either (i) a N-terminal pro-peptide fragment on the C-terminal end of said signal peptide and wherein said N-terminal pro-peptide fragment comprises a sorting motif consisting of the amino acid sequence DLXₓEX_{y}L (SEQ ID No. 36), where x is an integer from 1 to 20, y is an integer from 1 to 10, and each instance of X may independently be any amino acid (for the avoidance of doubt, this means that in the present invention in the first sequence of e.g. 1 to 20 X, each X may individually be any amino acid, and in the second sequence of e.g. 1 to 10 X, each X may individually be any amino acid, particularly as further defined herein), or wherein said pre-propeptide comprises (ii) a N-terminal pro-peptide fragment of a pre-pro-neuropeptide or protein sorted to large dense core vesicles, other than pre-prodynorphin, on the C-terminal end of said signal peptide and wherein said N-terminal pro-peptide fragment comprises a sorting motif of said pre-pro-neuropeptide or protein sorted to large dense core vesicles, and wherein said N-terminal pro-peptide fragment consists of 16 to 90 amino acids, and
   - wherein said pre-prodynorphyin or pre-prodynorphin-variants comprise at least one of the following sequences selected from the group:
      a. Dyn A that is SEQ ID No. or a variant thereof consisting of the first 13 amino acids from the N-terminal end or a variant thereof consisting of the first 8 amino acids from the N-terminal end
      b. Dyn B that is SEQ ID No. 3
      c. leumorphin that is SEQ ID No. 4
      d. variants of Dyn A, said variants having an amino acid sequence identity of at least 60 % within the first 8 amino acids from the N-terminal end of SEQ ID No. 2,
      e. variants of Dyn B, said variants having an amino acid sequence identity of at least 60 % within the first 8 amino acids from the N-terminal end of SEQ ID No. 3,
      f. variants of leumorphin, said variants having an amino acid sequence identity of at least 60 % within the first 8 amino acids from the N-terminal end of SEQ ID No. 4.
2. A delivery vector comprising a DNA sequence encoding pre-prodynorphin or pre-prodynorphin-variants according to embodiment 1, wherein said N-terminal pro-peptide fragment consists of 20 to 90 amino acids, preferably 30 and 90 amino acids.
3. A delivery vector comprising a DNA sequence encoding pre-prodynorphin or pre-prodynorphin-variants according to embodiment 1 or 2, wherein said N-terminal pro-peptide fragment on the C-terminal end of the signal peptide is a modified propeptide fragment of ppDyn wherein the unmodified propeptide fragment of ppDyn is 
SEQ ID No. 5

DCLSRCSLCA VKTQDGPKPI NPLICSLQCQ AALLPSEEWE RCQSFLSFFT
PSTLGLNDKE DLGSKSVGEG PYSELAKLSG SFLKELEKSK FLPSISTKEN
TLSKSLEEKL RGLSDGFREG AESELMRDAQ LNDGAMETGT LYLAEEDPKE
QV
   and wherein the modification of said propeptide fragment of ppDyn is a shortening;
   or the modification is a replacement of parts of SEQ ID No. 5 with a propeptide of or a fragment of a propeptide of a neuropeptide;
   wherein the modified propeptide fragment of ppDyn i) comprises at least one sorting motif consisting of the amino acid sequence DLXₓEX_{y}L (SEQ ID No. 36) or ii) comprises a sorting motif of said pre-pro-neuropeptide or protein sorted to large dense core vesicles other than pre-prodynorphin.
4. A delivery vector comprising a DNA sequence encoding pre-prodynorphin or pre-prodynorphin-variants according to any of embodiments 1 to 3, wherein said N-terminal pro-peptide fragment on the C-terminal end of the signal peptide is a modified propeptide fragment of ppDyn that comprises or consists of 
SEQ ID No. 6
DCLSRCSLCAVKTQDGPKPINPLICSLQCQAALLPSEEWERCQSFLSFFTPSTLG
LNDKEDLGSKSVGEGPYSELAKLSGSFLRKEQVKR
5. A delivery vector comprising a DNA sequence encoding pre-prodynorphin or pre-prodynorphin-variants according to any of embodiments 1 to 3, wherein said N-terminal pro-peptide fragment on the C-terminal end of the signal peptide is a modified propeptide fragment of ppDyn that comprises or consists of
   SEQ ID No. 7 
DLGSKSVGEG PYSELAKLSG SFLKELEKSK FLPSISTKEN TLSKSLEEKL
RGLSDGFREG AESELMRDAQ LNDGAMETGT LYLAEEDPKE QV
   or SEQ ID No. 8
      DLGSKSVGEG PYSELAKLSG SFLRKE QV
   or SEQ ID No 9
      DLGSKSVGEG PYSELAKLRKE QV.
6. A delivery vector comprising a DNA sequence encoding pre-prodynorphin or pre-prodynorphin-variants according to embodiment 3, wherein the modification is a replacement of parts of SEQ ID No. 5 with a propeptide of or a fragment of a propeptide of a neuropeptide; wherein the modified propeptide fragment of ppDyn i) comprises at least one sorting motif consisting of the amino acid sequence DLXxEXyL (SEQ ID No. 36) or ii) comprises a sorting motif of said pre-pro-neuropeptide or protein sorted to large dense core vesicles other than pre-prodynorphin.
7. A delivery vector comprising a DNA sequence encoding pre-prodynorphin or pre-prodynorphin-variants according to embodiment 6 wherein the modified propeptide fragment comprises or consists of SEQ ID No. 10
   MPRSCCSRSGALLLALLLQASMEVRGWCLESSQCQ**DL**TTESN**L**LECIRACKP
8. A delivery vector comprising a DNA sequence encoding pre-prodynorphin or pre-prodynorphin-variants according to embodiment 6 wherein the modified propeptide fragment comprises or consists of a propeptide fragment of preproEnkephalin, preproBDNF, preproTachykinin, prepro-Somatostatin, pre-pro-VIP, prepro-CCK, preproNociceptin or preproNPY, wherein said propeptide fragment comprises a sorting motif, in particular said propeptide fragment maybe selected from the group comprising of any of SEQ ID Nos: 37 to 52.
9. A delivery vector comprising a DNA sequence encoding pre-prodynorphin or pre-prodynorphin-variants according to embodiments 1-8 wherein the modified propeptide fragment is optionally flanked by peptidase recognition signals comprising K, R, KR, RK or RR.
10. A delivery vector comprising a DNA sequence encoding pre-prodynorphin or pre-prodynorphin-variants according to any of embodiments 1-9, wherein the target cell are neuronal cells of the central nervous system.
11. A delivery vector comprising a DNA sequence encoding pre-prodynorphin or pre-prodynorphin-variants according to any of embodiments 1-10, wherein the signal peptide is a peptide sequence of from 10 to 30 amino acids at the N-terminal end of precursor-proteins that are destined into the lumen of the endoplasmatic reticulum.
12. A delivery vector comprising a DNA sequence encoding pre-prodynorphin or pre-prodynorphin-variants according to any of embodiments 1-11, wherein the signal peptide is selected from the group comprising:
   MAWQGLVLAACLLMFPSTTA (SEQ ID No. 11)
   MARFLTLCTWLLLLGPGLLATVRA (SEQ ID No. 12)
   MLGNKRLGLSGLTLALSLLVCLGALAEA (SEQ ID No. 13)
   MLSCRLQCALAALSIVLALGCVTG (SEQ ID No. 14)
   MKILVALAVFFLVSTQLFA (SEQ ID No. 15)
   MRIMLLFTAILAFSLA (SEQ ID No. 16)
   MPRSCCSRSGALLLALLLQASMEVRG (SEQ ID No. 17)
   MNSGVCLCVLMAVLAAGA (SEQ ID No. 18)
   MKVLLCDLLLLSLFSSVFS (SEQ ID No. 19)
   MQPTLLLSLLGAVGLAAVNS (SEQ ID No. 20)
13. A delivery vector according to any of embodiments 1-12, wherein said delivery vector leads to release-on-demand of dynorphins or dynorphin-variants with agonistic effects on human Kappa Opioid Receptors.
14. A delivery vector according to any of embodiments 1-13, wherein the dynorphin variants have an amino acid sequence identity of at least 70 % within the first 8 amino acids from the N-terminal end of SEQ ID No. 2 (YGGFLRRI), SEQ ID No. 3 (YGGFLRRQ) or SEQ ID No. 4 (YGGFLRRQ), respectively.
15. A delivery vector according to any of embodiments 1-14, wherein the variants have an amino acid sequence identity of at least 80 % within the first 8 amino acids from the N-terminal end of SEQ ID No. 2, SEQ ID No. 3 or SEQ ID No. 4, respectively.
16. A delivery vector comprising a DNA sequence encoding pre-prodynorphin or pre-prodynorphin-variants according to any of embodiments 1 to 15, wherein said delivery vector comprises a DNA sequence encoding a dynorphin variant selected from the group comprising:
   a. SEQ ID No. 21 (YGZFZ₁RRZRZKLKWDNQ)
   b. SEQ ID No. 22 (YGZFZ₂RRZFZ₃VVT)
   c. SEQ ID No. 23 (YGZFZ₄RRZFZ₅VVTRSQEDPNAYSGELFDA),
   wherein each instance of Z, as well as any of Z₁ to Z₅ stands each independently for any of the naturally occurring amino acids, and wherein at least one Z in a sequence according to a.; b. or c. is substituted by another amino acid when compared to the wild-type sequence of said dynorphin fragment according to a sequence according SEQ ID No. 2, SEQ ID No. 3 or SEQ Id No. 4, respectively.
17. A delivery vector according to embodiment 16, wherein Z₁ to Z₅ is independently selected from the group comprising: alanine, glycine, asparagine, glutamine, leucine, histidine, methionine, serine, valine and isoleucine, and/or wherein each Z is independently selected from the group comprising alanine, glycine, asparagine, glutamine, leucine, serine, valine and isoleucine.
18. A delivery vector according to any of embodiments 1 to 17 , wherein said delivery vector comprises a DNA sequence encoding a pre-prodynorphin-variant that comprises at least one of the following sequences of variants selected from the group comprising:
   YGGFLRRQFKVVT (SEQ ID No. 3)
   YGAFLRRQFKVVT (SEQ ID No. 24)
   YGGFLRRAFKVVT (SEQ ID No. 25)
   YGGFLRRQFAVVT (SEQ ID No. 26)
   YGAFLRRAFKVVT (SEQ ID No. 27)
   YGAFLRRQFAVVT (SEQ ID No. 28)
   YGMFLRRQFKVVT (SEQ ID No. 29)
   YGGFSRRQFKVVT (SEQ ID No. 30)
   YGAFSRRQFKVVT (SEQ ID No. 31)
   YGAFLRRHFKVVT (SEQ ID No. 32)
   YGGFLRRHFKVVT (SEQ ID No. 33)
   YGMFSRRQFKVVT (SEQ ID No. 34)
   YGMFLRRHFKVVT (SEQ ID No. 35)
19. A delivery vector according to any of embodiments 1 to 18, wherein said delivery vector comprises in addition a recombinant adeno-associated virus (AAV) vector genome or a recombinant lentivirus genome.
20. A delivery vector according to any of embodiments 1 to 19 comprising a recombinant adeno-associated virus (AAV) vector genome comprising inverted terminal repeats (ITR) preferably derived from AAV serotype 2, alternatively from AAV serotype 1, 3, 4, 5, 6, 7, 8, 9, 10, rh10, 11, 12, 13, 14, or reengineered variants thereof, wherein said vector genome is packaged in an AAV capsid selected from the group comprising AAV serotypes 1, 2, 3, 4, 5, 6, 7, 8, 9, 10, rh10, 11, 12, 13, 14, serpentine AAV, ancestral AAV, AAV-TT, AAVv66, AAV1P4, AAV1P5, AAV-PHP.B, AAV-PHP.eB, AAV2-HBKO, AAV.CAP-B10, AAV.CAP-MAC, AAV2.NN, or further AAV capsid mutants derived thereof, preferably of AAV serotype 1 or 2.
21. A delivery vector comprising a DNA sequence encoding pre-prodynorphin or pre-prodynorphin-variants according to any of the preceding embodiments.
22. A recombinant virus particle or a liposome or nanoparticle, comprising a delivery vector according to any of the preceding embodiments.
23. The recombinant virus particle or liposome or nanoparticle of embodiment 22, wherein said delivery vector comprises in addition a recombinant adeno-associated virus (AAV) vector genome and said rAAV vector genome is encapsidated in an AAV capsid or wherein said delivery vector comprises in addition a recombinant lentivirus vector genome and is packaged in a lentivirus particle.
24. A delivery vector or recombinant virus particle or liposome or nanoparticle according to any of embodiments 1 to 23 for use in delivering a nucleic acid to a cell of the central nervous system, comprising contacting the cell with the delivery vector or recombinant virus particle or liposome or nanoparticle under conditions sufficient for the DNA sequence encoding pre-prodynorphin or pre-prodynorphin-variants to be introduced into the cell.
25. A delivery vector or recombinant virus particle or liposome or nanoparticle according to any of embodiments 1 to 24 for use as medicament.
26. A delivery vector or recombinant virus particle or liposome or nanoparticle according to any of embodiments 1 to 24 for use in treating focal epilepsy in a subject, in particular mesial temporal lobe epilepsy, or for use in preventing epileptic seizures in a subject that suffers from focal epilepsy whereby said delivery vector or recombinant virus particle or liposome or nanoparticle provides activation of human Kappa Opioid Receptors in the epileptogenic focus, thereby inhibiting seizures.
27. Peptide with agonistic effects on human Kappa Opioid Receptors (KOR) wherein said peptide is selected from the group comprising the peptides:
   SEQ ID No. 21 (YGZFZ₁RRZRZKLKWDNQ)
   SEQ ID No. 22 (YGZFZ₂RRZFZ₃VVT)
   SEQ Id No. 23 (YGZFZ₄RRZFZ₅VVTRSQEDPNAYSGELFDA
   wherein each instance of Z, as well as any of Z₁ to Z₅ stands each independently for any of the naturally occurring amino acids, and wherein at least one Z in a sequence according to a.; b. or c. is substituted by another amino acid when compared to the wild-type sequence of said dynorphin fragment according to a sequence according SEQ ID No 2, SEQ ID No. 3, SEQ ID No 4-
28. Peptide with agonistic effects on human Kappa Opioid Receptors (KOR) wherein said peptide is selected from the group comprising the peptides:
   YGAFLRRQFKVVT (SEQ ID No. 24)
   YGGFLRRAFKVVT (SEQ ID No. 25)
   YGGFLRRQFAVVT (SEQ ID No. 26)
   YGAFLRRAFKVVT (SEQ ID No. 27)
   YGAFLRRQFAVVT (SEQ ID No. 28)
   YGMFLRRQFKVVT (SEQ ID No. 29)
   YGGFSRRQFKVVT (SEQ ID No. 30)
   YGAFSRRQFKVVT (SEQ ID No. 31)
   YGAFLRRHFKVVT (SEQ ID No. 32)
   YGGFLRRHFKVVT (SEQ ID No. 33)
   YGMFSRRQFKVVT (SEQ ID No. 34)
   YGMFLRRHFKVVT (SEQ ID No. 35).

### Figure Description

**Figure 1**: Overview of the shortening of the human ppDyn cDNA.

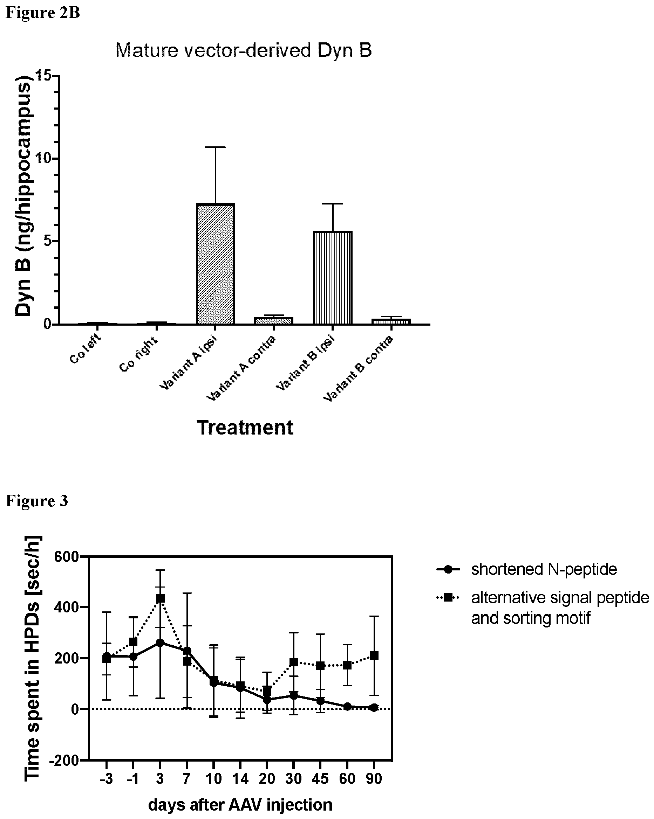

**Figure 2****:** Results of an ELISA to measure the content of (A) dynorphin A (DynA) and (B) dynorphin B (DynB) after intraparenchymal CNS transduction of AAV vectors expressing the indicated ppDyn variants. Variant A = shortened N-peptide. Variant B = signal and N-Peptide replaced by POMC. ipsi = site of AAV transduction, contra = non-transduced (control) site.
**Figure 3**: Seizure suppression by two scAAV vector variants containing shortened pDyn cDNA or with alternative signal and sorting sequence. Data represent N± SEM (N=3).

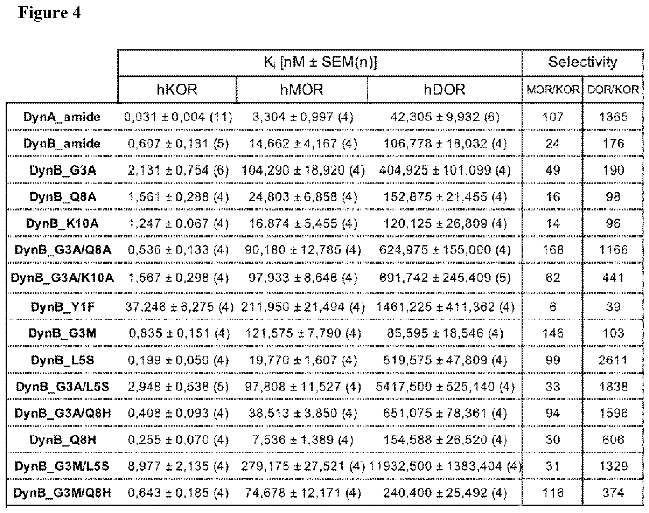

**Figure 4**: Testing affinity and selectivity of Dyn peptides for the three human opioid receptors: kappa (hKOR), mu (hMOR) and delta (hDOR) by radioligand displacement assays. Depicted is Dyn-binding to hKOR, hMOR and hDOR. The relative selectivity was evaluated by calculating the ratio of MOR and KOR, or the ratio of DOR and KOR. The Kᵢ is represented in nM ± SEM.

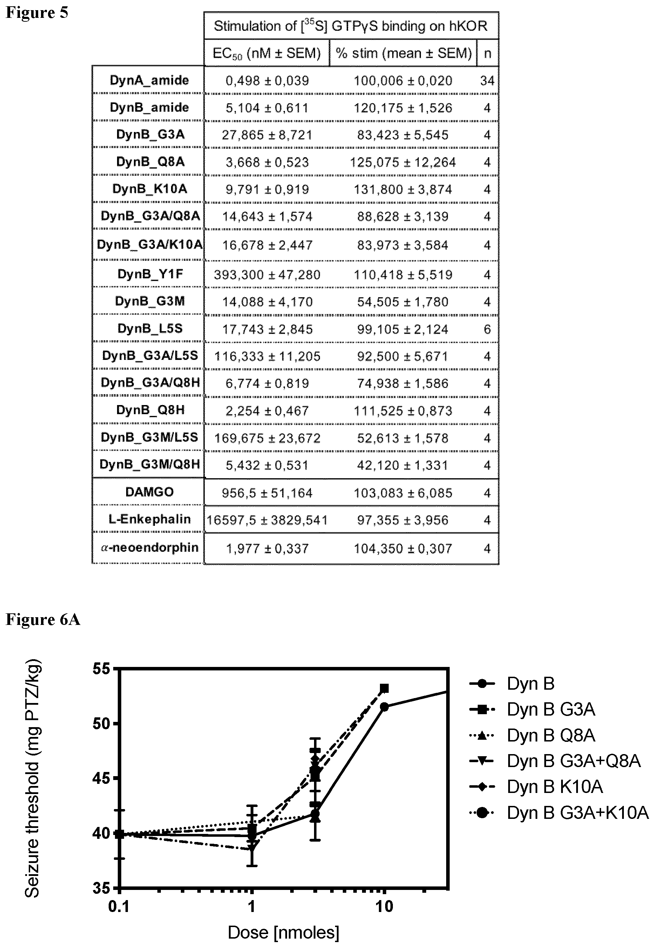

**Figure 5**: Measuring the activation effect of Dyn variants on KOR by quantification of the G-protein activation by using the GTPgammaS assay. The stimulation of [35^{S}] GTPyS binding on hKOR is indicated by the EC₅₀ and by calculation of percent stimulation. The EC₅₀ is depicted in nM ± SEM and the % stimulation is depicted in nM ± mean.

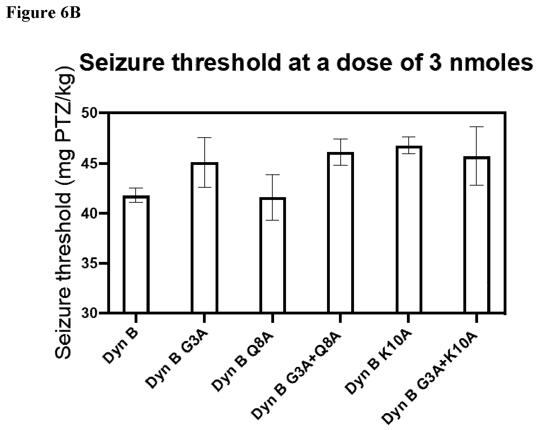

**Figure 6** **A and B**: Effect of DynB variants on induction of acute seizures. Figure 6A shows the seizure threshold at different doses from 0.1 to 10 nmoles for six depicted peptides. Figure 6B depicts the seizure threshold at a dose of 3 nmoles for unmodified DynB and five modified peptides.

### References

Ausubel, F. M. et al. (eds.), Current Protocols in Molecular Biology, Greene Publishing Associates, Wiley (1989)
Berns K.I. and C.R. Parrish in FIELDS VIROLOGY, eds. D.N. Knipe and P.M. Howley, volume 2, chapter 57 (6th ed., 2013, Wolters-Kluwer, Lippincott Williams & Wilkins Publishers).
Coatsworth JJ. Studies on the clinical effect of marketed antiepileptic drugs. 1971. NINDS Monograph 12
de Lanerolle NC,Williamson A,Meredith C,Kim JH,Tabuteau H,Spencer DD, Brines ML Dynorphin and the kappa 1 ligand [3h]u69,593 binding in the human epileptogenic hippocampus. 1997. Epilepsy Res 28:189-205.
Engel JJ. Mesial temporal lobe epilepsy: What have we learned? 2001. Neuroscientist 7:340-352.
Gambardella A,Manna I,Labate A,Chifari R,Serra P,La Russa A,LePiane E,Cittadella R,Andreoli V,Sasanelli F,Zappia M,Aguglia U, Quattrone A. Prodynorphin gene promoter polymorphism and temporal lobe epilepsy. 2003. Epilepsia 44:1255-1256.
Gossen M and Bujard H, (1992) Tight control of gene expression in mammalian cells by tetracyclin-responsive promoters. Proc. Natl. Acad. Sci. USA 89:5547-51.
Gossen M, Freundlieb S, Bender G, Müller G, Hillen W and Bujard H, (1995) Transcriptional activation by tetracyclines in mammalian cells. Science 268(5218):1766-9.
Grimm D, Kay MA, Kleinschmidt JA (2003) Helper virus-free, optically controllable, and two-plasmid-based production of adeno-associated virus vectors of serotypes 1 to 6, Mol Ther 7(6) 839-50
Harvey DM, Caskey CT (1998) Inducible control of gene expression: prospects for gene therapy. Curr. Opin. Chem. Biol. 2:512-8.
Henriksen SJ,Chouvet G,McGinty J, Bloom FE. Opioid peptides in the hippocampus: Anatomical and physiological considerations. 1982. Ann N Y Acad Sci 398:207-220.
Hüser D, Gogol-Döring A, Chen W, Heilbronn R (2014) Adeno-associated virus type 2 wild-type and vector-mediated genomic integration profiles in human diploid fibroblasts analyzed by 3rd generation PacBio DNA sequencing. J Virol l88 (19): 11253-11263
Loacker S,Sayyah M,Wittmann W,Herzog H, Schwarzer C. Endogenous dynorphin in epileptogenesis and epilepsy: Anticonvulsant net effect via kappa opioid receptors. 2007. Brain 130:1017-1028.
Loftus SK, Erickson RP, Walkley SU, Bryant MA, Incao A, Heidenreich RA, Pavan WJ (2002), Rescue of neurodegeneration in Niemann-Pick C mice by a prion promoter-driven Npc1 cDNA transgene. Hum. Mol. Genet. 11:3107-14.
Loscher W, Schmidt D. Modern antiepileptic drug development has failed to deliver: Ways out of the current dilemma. 2011. Epilepsia 52:657-678.
Magari SR Rivera VM, Iulicci JD, Gilman M, Cerasoli F Jr, (1997) Pharmacologic control of a humanized gene therapy system implanted into nude mice J. Clin. Invest. 100:2865-72
McCarty DM, Monahan PE, Samulski RJ (2001) Self-complementary recombinant adeno-associated virus (scAAV) vectors promote efficient transduction independently of DNA synthesis, Gene Ther 8(16) 1248-54
McNamara JO. Emerging insights into the genesis of epilepsy. 1999. Nature 399:A15-22.
Mietzsch M, Broecker F, Reinhardt A, Seeberger PH, Heilbronn R (2014) Differential adeno-associated virus serotype-specific interaction patterns with synthetic heparins and other glycans. J Virol 88: 2991-3003
Mietzsch M, Grasse S, Zurawski C, Weger S, Bennett A, Agbandje-McKenna M, Muzyczka N, Zolotukhin S, Heilbronn R (2014) OneBac: Platform for scalable and high-titer production of adeno-associated virus serotype 1-12 vectors for gene therapy. Hum Gene Ther 25: 212-222
Muzyczka (1992) Use of adeno-associated virus as a general transduction vector for mammalian cells. Curr. Topics Microbiol. Immunol. 158:97-129).
No D, Yao TP Evans RM (1996) Ecdysone-inducible gene expression in mammalian cells and transgenic mice Proc. Natl. Acad. Sci. USA 93:3346.-51
Pillay S, Meyer NL, Puschnik AS, Davulcu O, Diep J, Ishikawa Y, Jae LT, Wosen JE, Nagamine CM, Chapman MS, Carette JE (2016) Nature 530 (7588) 108-12.
Pirker S,Gasser E,Czech T,Baumgartner C,Schuh E,Feucht M,Novak K,Zimprich F, Sperk G. Dynamic up-regulation of prodynorphin transcription in temporal lobe epilepsy. 2009. Hippocampus 19:1051-1054.
Rosenzweig A (2007), Vectors for Gene Therapy. In: Current Protocols in Human Genetics. Wiley John and Sons, Inc.: DOI: 10.1002/0471142905.hg1200s52.
Schunk E,Aigner C,Stefanova N,Wenning G,Herzog H, Schwarzer C. Kappa opioid receptor activation blocks progressive neurodegeneration after kainic acid injection. 2011. Hippocampus 21:1010-1020.
Schwarzer C. 30 years of dynorphins--new insights on their functions in neuropsychiatric diseases. 2009. Pharmacol Ther 123:353-370.
Siggins GR,Henriksen SJ,Chavkin C, Gruol D. Opioid peptides and epileptogenesis in the limbic system: Cellular mechanisms. 1986. Adv Neurol 44:501-512.
Simonato M, Romualdi P. Dynorphin and epilepsy. 1996. Prog Neurobiol 50:557-583.
Solbrig MV,Adrian R,Chang DY, Perng GC. Viral risk factor for seizures: Pathobiology of dynorphin in herpes simplex viral (hsv-1) seizures in an animal model. 2006. Neurobiol Dis 23:612-620.
Spencer S, Huh L. Outcomes of epilepsy surgery in adults and children. 2008. Lancet Neurol 7:525-537.
Stogmann E,Zimprich A,Baumgartner C,Aull-Watschinger S,Hollt V, Zimprich F. A functional polymorphism in the prodynorphin gene promotor is associated with temporal lobe epilepsy. 2002. Ann Neurol 51:260-263.
Stutika C, Gogol-Doring A, Botschen L, Mietzsch M, Weger S, Feldkamp M, Chen W, Heilbronn R (2015) A comprehensive RNA-Seq analysis of the adeno-associated virus type 2 transcriptome reveals novel AAV transcripts, splice variants, and derived proteins. J Virol 90(3) 1278-89
Takahashi M,Senda T,Tokuyama S, Kaneto H. Further evidence for the implication of a kappa-opioid receptor mechanism in the production of psychological stress-induced analgesia. 1990. Jpn J Pharmacol 53:487-494.
Tani M, Fuentes ME, Petersen JW, Trapp BD, Durham SK, Loy JK, Bravo R, Ransohoff RM, Lira SA (1996) Neutrophil infiltration, glial reaction, and neurological disease in transgenic mice expressing the chemokine N51/KC in oligodendrocytes. J. Clin. Invest. 98:529-39.
Toll, L., Berzetei-Gurske, I. P., Polgar, W. E., Brandt, S. R., Adapa, I. D., Rodriguez, L., et al. (1998). Standard binding and functional assays related to medications development division testing for potential cocaine and opiate narcotic treatment medications. NIDA Res Monogr 178, 440-466.
Tortella FC. Endogenous opioid peptides and epilepsy: Quieting the seizing brain? 1988. Trends Pharmacol Sci 9:366-372.
Wang Y, DeMayo FJ, Tsai SY, O'Malley BW (1997) Ligand-inducible and liver-specific target gene expression in transgenic mice. Nat. Biotech. 15:239-43
Wang Y, Xu J, Pierson T, O'Malley BW, Tsai SY (1997) Positive and negative regulation of gene expression in eucaryotic cells with an inducible transcriptional regulator. Gene Ther, 4:432-41.
Melanie Widmann, Andreas Lieb, Angela Steck, Barbara Fogli, Anna Mutti, Christoph Schwarzern doi: https://doi.org/10.1101/2022.07.05.498820
Xiao X, Li J, Samulski RJ (1998) Production of high-titer recombinant adeno-associated virus vectors in the absence of helper adenovirus, JVirol 72(3) 2224-32
Zangrandi L, Burtscher J, MacKay J, Colmers W, & ∗Schwarzer C∗ (2016) The G-protein biased partial kappa opioid receptor agonist 6'-GNTI blocks hippocampal paroxysmal discharges without inducing aversion. British J Pharmacol, 173(11):1756-67,doi: 10.1111/bph.13475

### Examples

### Example 1

Shortening of the human ppDyn cDNA to enable packing into scAAV vectors. Some amino acids in the region between signal peptide and the region coding for active peptides can be removed. However, a sorting motif responsible for packing the propeptide into large dense core vesicles needs to be conserved. Alternatively, the entire part N-terminal to the region coding for active peptides can be replaced by a different signal peptide and sorting motif.

### Example 2

### Production of mature dynorphins by two scAAV vector variants

Production of mature dynorphins by two scAAV vector variants containing shortened pDyn cDNA. The vectors were injected into the dorsal hippocampus of naive wild-type mice. After 2 weeks the hippocampi were resected and the content of dynorphin A (DynA) and dynorphin B (DynB) was measured by ELISA as described in Agostinho et al. (2019), see Figure 2. The production of mature peptides occurs only in large dense core vesicles, proving the correctness of sorting of the shortened pDyn.

### Example 3

### Seizure suppression by shortened pDyn cDNA

Seizure suppression by two scAAV vector variants containing shortened pDyn cDNA. The vectors were injected into the dorsal hippocampus of epileptic wild-type mice. EEGs were recorded and analyzed for hippocampal paroxysmal discharges (HPD), representing drug-resistant focal seizures. The treatment of animals with kainic acid, the implantation of electrodes and the analysis is described in Widmann et al. (2022).

### Example 4

### Radio Binding Assay to determine affinity and selectivity

A radio ligand binding assay was used to determine the affinities of unmodified and modified dynorphin peptides to the 3 human classical opioid receptors: kappa (hKOR), mu (hMOR) and delta (hDOR). Besides the affinity to the receptor, also the selectivity of hKOR vs. the other OR receptors was calculated.

Competitive heterologous binding assays were conducted on hKOR-CHO, hMOR-CHO, hDOR-CHO cell membranes. Membranes were prepared in 50 mM Tris-HCl buffer pH 7.7. Cells were harvested by scraping the plates with a rubber policeman and then centrifuged at 500 x g for 10 min. The cell pellet was resuspended in Tris-HCl, homogenized with a Dounce homogenizer and centrifuged at 27000 x g for 15 min. The pellets were resuspended in Tris-HCl and homogenized through a 27G needle. The homogenate was stored at -70 °C until use. Binding assays were conducted using [3H]-U69,593, [3H]-DAMGO and [3H]-Diprenorphine for labelling κ, µ and δ opioid receptors. Non-specific binding was determined by using 1 µM of the unlabeled counterpart of each radioligand. Cell membranes (10 to 30 µg) were incubated with the appropriate radioligand and increasing concentration of test peptide, in a total volume of 1 mL Tris-HCl, for 60 min at 25 °C. After incubation, reactions were terminated by three rapid washes with Tris-HCl and filtration through glass fiber filters (GF/C Whatman) on a Brandel M-24 cell harvester. The bound radioactivity was measured by liquid scintillation (Carl Roth GmbH, Karlsruhe, Germany) counting on a Packard 1600 TR Tri-Carb Beta counter (Canberra, Belgium). The results are depicted in Figure 4.

### Example 5

### GTPyS for hKOR

The [35S]GTPyS functional assay was used to determine the potency and efficacy of unmodified and modified dynorphin variants to activate the G-protein through interaction with either hKOR, hMOR or hDOR.

Functional assays were conducted on hKOR-CHO, hMOR-CHO, hDOR-CHO cell membranes. Membranes were prepared in a buffer containing 20 mM HEPES, 10 µM GDP, cell membrane (10 to 30 µg) and 0.05 nM [35S]GTPyS. The buffer containing the cell membranes was incubated with increasing concentration of test peptide, in a total volume of 1 mL, for 60 min at 25 °C. Non-specific binding was determined using unlabeled 10 µM GTPyS. Samples were rapidly washed three times with Tris-HCl and filtered through glass fiber filters (GF/B Whatman). The bound radioactivity was measured by liquid scintillation (Carl Roth GmbH, Karlsruhe, Germany) counting on a Packard 1600 TR Tri-Carb Beta counter (Canberra, Belgium). The results are shown in Figure 5.

### Example 6

### Effect of Dyn Variants on Induction of Acute Seizures

The influence of modified and unmodified dynorphins on the seizure threshold of dynorphin deficient mice was tested by PTZ tail-vein infusion, see Figures 6A to B. While all peptides induced a comparable maximal effect, some displayed increased potency.

Peptides were dissolved in saline and injected intracisternally under mild sevoflurane anesthesia. Seizure threshold was measured as described in Loacker et al. (2007) 30 minutes after peptide injection.

## Claims

1. A delivery vector comprising a DNA sequence encoding pre-prodynorphin or pre-prodynorphin-variants and
• wherein said delivery vector drives expression of a pre-propeptide in a target cell, and
• wherein said delivery vector comprising said DNA sequence enables the release of dynorphin or dynorphin-variants from the target cell on demand, and
• wherein said pre-propeptide is pre-prodynorphin or a pre-prodynorphin-variant and
• wherein said pre-propeptide comprises a signal peptide, wherein the signal peptide is a N-terminal extension of a nascent polypeptide chain and wherein said signal peptide mediates protein targeting to the lumen of the endoplasmatic reticulum, and,
• wherein said pre-propeptide comprises either (i) a N-terminal pro-peptide fragment on the C-terminal end of said signal peptide and wherein said N-terminal pro-peptide fragment comprises a sorting motif consisting of the amino acid sequence DLXₓEX_{y}L (SEQ ID No. 36), where x is an integer from 1 to 20, y is an integer from 1 to 10, and each instance of X may independently be any amino acid (for the avoidance of doubt, this means that in the present invention in the first sequence of e.g. 1 to 20 X, each X may individually be any amino acid, and in the second sequence of e.g. 1 to 10 X, each X may individually be any amino acid, particularly as further defined herein), or wherein said pre-propeptide comprises (ii) a N-terminal pro-peptide fragment of a pre-pro-neuropeptide or protein sorted to large dense core vesicles, other than pre-prodynorphin, on the C-terminal end of said signal peptide and wherein said N-terminal pro-peptide fragment comprises a sorting motif of said pre-pro-neuropeptide or protein sorted to large dense core vesicles, and wherein said N-terminal pro-peptide fragment consists of 16 to 90 amino acids, and
• wherein said pre-prodynorphyin or pre-prodynorphin-variants comprise at least one of the following sequences selected from the group:
a. Dyn A that is SEQ ID No. 2 or a variant thereof consisting of the first 13 amino acids from the N-terminal end or a variant thereof consisting of the first 8 amino acids from the N-terminal end
b. Dyn B that is SEQ ID No. 3
c. leumorphin that is SEQ ID No. 4
d. variants of Dyn A, said variants having an amino acid sequence identity of at least 60 % within the first 8 amino acids from the N-terminal end of SEQ ID No. 2,
e. variants of Dyn B, said variants having an amino acid sequence identity of at least 60 % within the first 8 amino acids from the N-terminal end of SEQ ID No. 3,
f. variants of leumorphin, said variants having an amino acid sequence identity of at least 60 % within the first 8 amino acids from the N-terminal end of SEQ ID No. 4.

2. A delivery vector comprising a DNA sequence encoding pre-prodynorphin or pre-prodynorphin-variants according to claim 1, wherein said N-terminal pro-peptide fragment consists of 20 to 90 amino acids, preferably 30 and 90 amino acids.

3. A delivery vector comprising a DNA sequence encoding pre-prodynorphin or pre-prodynorphin-variants according to claim 1 or 2, wherein said N-terminal pro-peptide fragment on the C-terminal end of the signal peptide is a modified propeptide fragment of ppDyn wherein the unmodified propeptide fragment of ppDyn is 
SEQ ID No. 5
DCLSRCSLCA VKTQDGPKPI NPLICSLQCQ AALLPSEEWE RCQSFLSFFT
PSTLGLNDKE DLGSKSVGEG PYSELAKLSG SFLKELEKSK FLPSISTKEN
TLSKSLEEKL RGLSDGFREG AESELMRDAQ LNDGAMETGT LYLAEEDPKE
QV
and wherein the modification of said propeptide fragment of ppDyn is a shortening;
or the modification is a replacement of parts of SEQ ID No. 5 with a propeptide of or a fragment of a propeptide of a neuropeptide;
wherein the modified propeptide fragment of ppDyn i) comprises at least one sorting motif consisting of the amino acid sequence DLXₓEX_{y}L (SEQ ID No. 36) or ii) comprises a sorting motif of said pre-pro-neuropeptide or protein sorted to large dense core vesicles other than pre-prodynorphin.

4. A delivery vector comprising a DNA sequence encoding pre-prodynorphin or pre-prodynorphin-variants according to any of claims 1 to 3, wherein said N-terminal pro-peptide fragment on the C-terminal end of the signal peptide is a modified propeptide fragment of ppDyn that comprises or consists of

SEQ ID No. 6
DCLSRCSLCAVKTQDGPKPINPLICSLQCQAALLPSEEWERCQSFLSFFTPSTLG
LNDKEDLGSKSVGEGPYSELAKLSGSFLRKEQVKR

5. A delivery vector comprising a DNA sequence encoding pre-prodynorphin or pre-prodynorphin-variants according to any of claims 1 to 3, wherein said N-terminal pro-peptide fragment on the C-terminal end of the signal peptide is a modified propeptide fragment of ppDyn that comprises or consists of
SEQ ID No. 7 
DLGSKSVGEG PYSELAKLSG SFLKELEKSK FLPSISTKEN TLSKSLEEKL
RGLSDGFREG AESELMRDAQ LNDGAMETGT LYLAEEDPKE QV
or SEQ ID No. 8
DLGSKSVGEG PYSELAKLSG SFLRKE QV
or SEQ ID No 9
DLGSKSVGEG PYSELAKLRKE QV

6. A delivery vector comprising a DNA sequence encoding pre-prodynorphin or pre-prodynorphin-variants according to claim 3, wherein the modification is a replacement of parts of SEQ ID No. 5 with a propeptide of or a fragment of a propeptide of a neuropeptide; wherein the modified propeptide fragment of ppDyn i) comprises at least one sorting motif consisting of the amino acid sequence DLXxEXyL (SEQ ID No. 36) or ii) comprises a sorting motif of said pre-pro-neuropeptide or protein sorted to large dense core vesicles other than pre-prodynorphin.

7. A delivery vector comprising a DNA sequence encoding pre-prodynorphin or pre-prodynorphin-variants according to claim 6 wherein the modified propeptide fragment comprises or consists of SEQ ID No. 10 MPRSCCSRSGALLLALLLQASMEVRGWCLESSQCQDLTTESNLLECIRACKP

8. A delivery vector comprising a DNA sequence encoding pre-prodynorphin or pre-prodynorphin-variants according to claim 6 wherein the modified propeptide fragment comprises or consists of a propeptide fragment of preproEnkephalin, preproBDNF, preproTachykinin, prepro-Somatostatin, pre-pro-VIP, prepro-CCK, preproNociceptin or preproNPY, wherein said propeptide fragment comprises said sorting motif, in particularly said propeptide fragment maybe selected from the group comprising of any of SEQ ID Nos: 37 to 52.

9. A delivery vector comprising a DNA sequence encoding pre-prodynorphin or pre-prodynorphin-variants according to any of claims 1-8 wherein the modified propeptide fragment is optionally flanked by peptidase recognition signals comprising K, R, KR, RK or RR.

10. A delivery vector comprising a DNA sequence encoding pre-prodynorphin or pre-prodynorphin-variants according to any of claims 1-9, wherein the target cells are neuronal cells of the central nervous system.

11. A delivery vector comprising a DNA sequence encoding pre-prodynorphin or pre-prodynorphin-variants according to any of claims 1-10, wherein the signal peptide is a peptide sequence of from 10 to 30 amino acids at the N-terminal end of precursor-proteins that are destined into the lumen of the endoplasmatic reticulum.

12. A delivery vector comprising a DNA sequence encoding pre-prodynorphin or pre-prodynorphin-variants according to any of claims 1-11, wherein the signal peptide is selected from the group comprising:
MAWQGLVLAACLLMFPSTTA (SEQ ID No. 11)
MARFLTLCTWLLLLGPGLLATVRA (SEQ ID No. 12)
MLGNKRLGLSGLTLALSLLVCLGALAEA (SEQ ID No. 13)
MLSCRLQCALAALSIVLALGCVTG (SEQ ID No. 14)
MKILVALAVFFLVSTQLFA (SEQ ID No. 15)
MRIMLLFTAILAFSLA (SEQ ID No. 16)
MPRSCCSRSGALLLALLLQASMEVRG (SEQ ID No. 17)
MNSGVCLCVLMAVLAAGA (SEQ ID No. 18)
MKVLLCDLLLLSLFSSVFS (SEQ ID No. 19)
MQPTLLLSLLGAVGLAAVNS (SEQ ID No. 20)

13. A delivery vector according to any of claims 1-12, wherein said delivery vector leads to release-on-demand of dynorphins or dynorphin-variants with agonistic effects on human Kappa Opioid Receptors.

14. A delivery vector according to any of claims 1-13, wherein the dynorphin variants have an amino acid sequence identity of at least 70 % within the first 8 amino acids from the N-terminal end of SEQ ID No. 2 (YGGFLRRI), SEQ ID No. 3 (YGGFLRRQ) or SEQ ID No. 4 (YGGFLRRQ), respectively.

15. A delivery vector according to any of claims 1-14, wherein the variants have an amino acid sequence identity of at least 80 % within the first 8 amino acids from the N-terminal end of SEQ ID No. 2, SEQ ID No. 3 or SEQ ID No. 4, respectively.

16. A delivery vector comprising a DNA sequence encoding pre-prodynorphin or pre-prodynorphin-variants according to any of claims 1 to 15, wherein said delivery vector comprises a DNA sequence encoding a dynorphin variant selected from the group comprising:
d. SEQ ID No. 21 (YGZFZ₁RRZRZKLKWDNQ)
e. SEQ ID No. 22 (YGZFZ₂RRZFZ₃VVT)
f. SEQ ID No. 23 (YGZFZ₄RRZFZ₅VVTRSQEDPNAYSGELFDA),
wherein each instance of Z, as well as any of Z₁ to Z₅ stands each independently for any of the naturally occurring amino acids, and wherein at least one Z in a sequence according to a.; b. or c. is substituted by another amino acid when compared to the wild-type sequence of said dynorphin fragment according to a sequence according SEQ ID No. 2, SEQ ID No. 3 or SEQ Id No. 4, respectively.

17. A delivery vector according to claim 16, wherein Z₁ to Z₅ is independently selected from the group comprising: alanine, glycine, asparagine, glutamine, leucine, histidine, methionine, serine, valine and isoleucine, and/or wherein each Z is independently selected from the group comprising alanine, glycine, asparagine, glutamine, leucine, serine, valine and isoleucine.

18. A delivery vector according to any of claims 1 to 17 , wherein said delivery vector comprises a DNA sequence encoding a pre-prodynorphin-variant that comprises at least one of the following sequences of variants selected from the group comprising:
YGGFLRRQFKVVT (SEQ ID No. 3)
YGAFLRRQFKVVT (SEQ ID No. 24)
YGGFLRRAFKVVT (SEQ ID No. 25)
YGGFLRRQFAVVT (SEQ ID No. 26)
YGAFLRRAFKVVT (SEQ ID No. 27)
YGAFLRRQFAVVT (SEQ ID No. 28)
YGMFLRRQFKVVT (SEQ ID No. 29)
YGGFSRRQFKVVT (SEQ ID No. 30)
YGAFSRRQFKVVT (SEQ ID No. 31)
YGAFLRRHFKVVT (SEQ ID No. 32)
YGGFLRRHFKVVT (SEQ ID No. 33)
YGMFSRRQFKVVT (SEQ ID No. 34)
YGMFLRRHFKVVT (SEQ ID No. 35)

19. A delivery vector according to any of claims 1 to 18, wherein said delivery vector comprises in addition a recombinant adeno-associated virus (AAV) vector genome or a recombinant lentivirus genome.

20. A delivery vector according to any of claims 1 to 19 comprising a recombinant adeno-associated virus (AAV) vector genome comprising inverted terminal repeats (ITR) preferably derived from AAV serotype 2, alternatively from AAV serotype 1, 3, 4, 5, 6, 7, 8, 9, 10, rh10, 11, 12, 13, 14, or reengineered variants thereof, wherein said vector genome is packaged in an AAV capsid selected from the group comprising AAV serotypes 1, 2, 3, 4, 5, 6, 7, 8, 9, 10, rh10, 11, 12, 13, 14, serpentine AAV, ancestral AAV, AAV-TT, AAVv66, AAV1P4, AAV1P5, AAV-PHP.B, AAV-PHP.eB, AAV2-HBKO, AAV. CAP-B10, AAV.CAP-MAC, AAV2.NN, or further AAV capsid mutants derived thereof, preferably of AAV serotype 1 or 2.

21. A delivery vector comprising a DNA sequence encoding pre-prodynorphin or pre-prodynorphin-variants according to any of the preceding claims.

22. A recombinant virus particle or a liposome or nanoparticle, comprising a delivery vector according to any of the preceding claims.

23. The recombinant virus particle or liposome or nanoparticle of claim 22, wherein said delivery vector comprises in addition a recombinant adeno-associated virus (AAV) vector genome and said rAAV vector genome is encapsidated in an AAV capsid or wherein said delivery vector comprises in addition a recombinant lentivirus vector genome and is packaged in a lentivirus particle.

24. A delivery vector or recombinant virus particle or liposome or nanoparticle according to any of claims 1 to 23 for use in delivering a nucleic acid to a cell of the central nervous system, comprising contacting the cell with the delivery vector or recombinant virus particle or liposome or nanoparticle under conditions sufficient for the DNA sequence encoding pre-prodynorphin or pre-prodynorphin-variants to be introduced into the cell.

25. A delivery vector or recombinant virus particle or liposome or nanoparticle according to any of claims 1 to 24 for use as medicament.

26. A delivery vector or recombinant virus particle or liposome or nanoparticle according to any of claims 1 to 24 for use in treating focal epilepsy in a subject, in particular mesial temporal lobe epilepsy, or for use in preventing epileptic seizures in a subject that suffers from focal epilepsy whereby said delivery vector or recombinant virus particle or liposome or nanoparticle provides activation of human Kappa Opioid Receptors in the epileptogenic focus, thereby inhibiting seizures.

27. Peptide with agonistic effects on human Kappa Opioid Receptors (KOR) wherein said peptide is selected from the group comprising the peptides:
SEQ ID No. 21 (YGZFZ₁RRZRZKLKWDNQ)
SEQ ID No. 22 (YGZFZ₂RRZFZ₃VVT)
SEQ Id No. 23 (YGZFZ₄RRZFZ₅VVTRSQEDPNAYSGELFDA
wherein each instance of Z, as well as any of Z₁ to Z₅ stands each independently for any of the naturally occurring amino acids, and wherein at least one Z in a sequence according to a.; b. or c. is substituted by another amino acid when compared to the wild-type sequence of said dynorphin fragment according to a sequence according SEQ ID No 2, SEQ ID No. 3, SEQ ID No 4-

28. Peptide with agonistic effects on human Kappa Opioid Receptors (KOR) wherein said peptide is selected from the group comprising the peptides:
YGAFLRRQFKVVT (SEQ ID No. 24)
YGGFLRRAFKVVT (SEQ ID No. 25)
YGGFLRRQFAVVT (SEQ ID No. 26)
YGAFLRRAFKVVT (SEQ ID No. 27)
YGAFLRRQFAVVT (SEQ ID No. 28)
YGMFLRRQFKVVT (SEQ ID No. 29)
YGGFSRRQFKVVT (SEQ ID No. 30)
YGAFSRRQFKVVT (SEQ ID No. 31)
YGAFLRRHFKVVT (SEQ ID No. 32)
YGGFLRRHFKVVT (SEQ ID No. 33)
YGMFSRRQFKVVT (SEQ ID No. 34)
YGMFLRRHFKVVT (SEQ ID No. 35).
